(19) Europäisches Patentamt
European Patent Office
Office européen des brevets

(11) EP 4 772 534 A1

(12) EUROPEAN PATENT APPLICATION
published in accordance with Art. 153(4) EPC

(43) Date of publication:
08.07.2026 Bulletin 2026/28

(21) Application number: 24859876.5

(22) Date of filing: 29.08.2024

(51) International Patent Classification (IPC):
C07K 16/00 (2006.01)          A61K 39/395 (2006.01)
A61K 47/54 (2017.01)          A61P 37/02 (2006.01)
C12N 1/15 (2006.01)          C12N 1/19 (2006.01)
C12N 1/21 (2006.01)          C12N 5/10 (2006.01)
C12N 15/13 (2006.01)          C12N 15/63 (2006.01)
C12P 21/08 (2006.01)

(52) Cooperative Patent Classification (CPC):
A61K 39/395; A61K 47/54; A61P 37/02;
C07K 16/00; C12N 5/10; C12N 15/63; C12N 15/74;
C12N 15/80; C12N 15/81

(86) International application number:
PCT/JP2024/030889

(87) International publication number:
WO 2025/047847 (06.03.2025 Gazette 2025/10)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 30.08.2023 JP 2023139802

(71) Applicant: Astellas Pharma, Inc.
Chuo-ku
Tokyo 103-8411 (JP)

(72) Inventors:
• YAMAJUKU, Daisuke
Tokyo 1038411 (JP)
• MIMASU, Shinya
Tokyo 1038411 (JP)
• TOMIMOTO, Yusuke
Tokyo 1038411 (JP)

(74) Representative: Hoffmann Eitle
Patent- und Rechtsanwälte PartmbB
Arabellastraße 30
81925 München (DE)

Remarks:
The complete document including Reference
Table(s) and the Sequence Listing(s) can be
downloaded from the EPO website

(54) Fc REGION VARIANT

(57) According to one embodiment, provided is a sialylated Fc region variant comprising the following amino acid variations (1) and (2): (1) amino acid residues 234 and/or 235 are substituted by serine, threonine, asparagine, glutamine, cysteine, aspartic acid, glutamic acid, lysine, arginine, histidine, glycine, alanine, proline, methionine, valine, or isoleucine; and (2) amino acid residue 293 or 294 is deleted.

EP 4 772 534 A1

**Description**

Technical Field

The present invention relates to an Fc region variant, a sialylated Fc region variant, a polypeptide comprising the sialylated Fc region variant, and a pharmaceutical composition comprising the polypeptide, etc.

Background Art

In recent years, antibody drugs have been actively developed and have also attracted a lot of interest. For example, intravenous immunoglobulin (IVIG) using an IgG antibody is used in the treatment of autoimmune diseases and inflammatory diseases, such as idiopathic thrombocytopenic purpura (ITP), Kawasaki disease, and Guillain-Barre syndrome (GBS) (Non Patent Literature 1). In IVIG, for example, polyclonal IgG antibodies are isolated from pooled plasma of several thousands or more of healthy donors, prepared for treatment, and used. However, the antibody drugs still present various problems. Examples thereof include generally large doses of the antibody drugs, high production cost, and possible production of anti-drug antibodies. If an antibody drug having high therapeutic effects can be developed, it is expected that effects equivalent to or greater than previous ones can be obtained even at decreased doses.

One of the factors that contribute to the therapeutic effects of various antibodies is sialic acid. In this context, the sialic acid, also called N-acetylneuraminic acid (Neu5Ac), is linked to a galactose residue in an Fc region to constitute a non-reducing end of IgG Fc N-glycan. Such sialylation in the IgG Fc region is known, for example, to acquire an anti-inflammatory effect; to obtain a better effect with increase in sialylation rate (Non Patent Literature 2); and to suppress B cells by a sugar chain having a sialic acid added thereto in IVIG preparations via CD22 (also called Siglec-2) (Non Patent Literature 3).

Hence, the sialylation of an immunoglobulin Fc region has received attention. A polypeptide containing an Fc region variant with an increased amount of sialic acid added is known to have an increased half-life and to have preferred anti-inflammatory characteristics (Patent Literature 1). A method of allowing host cells that exhibit β galactosyltransferase and sialyltransferase activities to express an antibody having mutations in an Fc region (e.g., F243, V264, and D265) is known as a method for increasing the amount of sialic acid added in an Fc region (Patent Literature 1). Also, studies have been made on a method for increasing the amount of sialic acid added by mutating specific amino acids (e.g., amino acids at positions 240 to 243, 258 to 267, and 290 to 305) of an Fc region using HEK293 and YB2/0 cells (Patent Literature 2).

Citation List

Patent Literature

   Patent Literature 1: International Publication No. WO 2012/113863
   Patent Literature 2: Japanese Translation of PCT International Application Publication No. 2017-522040

Non Patent Literature

   Non Patent Literature 1: Journal of Allergy and Clinical Immunology, Mar. 2017, Vol. 139 (3), Supplement, p. S1-S46
   Non Patent Literature 2: Science, Aug. 4, 2006, Vol. 313 (5787), p. 670-673
   Non Patent Literature 3: Blood, Sep. 9, 2010, Vol. 116 (10), p. 1698-1704

Summary of Invention

Technical Problem

An object of the present invention is to provide a sialylated Fc region variant with a further increased amount of sialic acid added.

Solution to Problem

The present inventors have conducted diligent studies and consequently found that many sialic acid molecules are added to an immunoglobulin Fc region to be sialylated, by adding specific amino acid variations to the Fc region.

The present invention is, for example, as follows.

[1] A sialylated Fc region variant comprising the following amino acid variations (1) and (2):

(1)

(a) amino acid residues 234 and/or 235 are substituted by serine, threonine, asparagine, glutamine, cysteine, aspartic acid, glutamic acid, lysine, arginine, histidine, glycine, alanine, proline, methionine, valine, or isoleucine, wherein the amino acids at positions 234 and 235 are the same or different after substitution;
(b) amino acid residue 234 is substituted by phenylalanine, tyrosine, tryptophane, isoleucine, valine, methionine, proline, alanine, or glycine, and amino acid residue 235 is substituted by serine, threonine, asparagine, glutamine, cysteine, aspartic acid, glutamic acid, lysine, arginine, or histidine; or
(c) amino acid residue 234 is substituted by serine, threonine, asparagine, glutamine, cysteine, aspartic acid, glutamic acid, lysine, arginine, or histidine, and amino acid residue 235 is substituted by phenylalanine, tyrosine, tryptophane, isoleucine, valine, methionine, proline, alanine, or glycine; and

(2) amino acid residue 293 or 294 is deleted,
wherein the amino acid residue numbers are amino acid positions according to EU index.

[2] The sialylated Fc region variant according to [1], wherein amino acid residues 234 and 235 are substituted by serine, threonine, asparagine, glutamine, cysteine, aspartic acid, glutamic acid, lysine, arginine, histidine, glycine, alanine, proline, methionine, valine, or isoleucine, wherein the amino acids at positions 234 and 235 are the same or different after substitution.

[3] The sialylated Fc region variant according to [1] or [2], wherein

amino acid residues 234 and 235 are substituted by alanine, or
amino acid residue 234 is substituted by alanine, and amino acid residue 235 is substituted by glutamic acid.

[4] The sialylated Fc region variant according to any of [1] to [3], wherein the Fc region is derived from human IgG.

[5] The sialylated Fc region variant according to [4], wherein the Fc region is derived from human Igγ1, human Igγ2, human Igγ3, or human Igγ4.

[6] The sialylated Fc region variant according to [4], wherein the sialylated Fc region variant comprises an amino acid sequence 90% or more identical to a sequence consisting of amino acids at positions 218 to 448 in the amino acid sequence represented by SEQ ID NO: 7 or an amino acid sequence 90% or more identical to a sequence consisting of amino acids at positions 217 to 447 in the amino acid sequence represented by SEQ ID NO: 13.

[7] A polypeptide comprising the sialylated Fc region variant according to any of [1] to [6].

[8] The polypeptide according to [7], wherein the polypeptide comprising the sialylated Fc region variant is an antibody.

[9] The polypeptide according to [8], wherein the antibody is an IgG antibody.

[10] The polypeptide according to [7], wherein the polypeptide comprising the sialylated Fc region variant is an Fc fusion protein molecule.

[11] The polypeptide according to [10], wherein the sialylated Fc fusion protein molecule comprises a non-human derived protein.

[12] A polynucleotide encoding the polypeptide according to any of [7] to [11].

[13] An expression vector comprising the polynucleotide according to [12].

[14] A host cell transformed with the expression vector according to [13].

[15] A method for producing a polypeptide comprising a sialylated Fc region variant, the method comprising the step of culturing the host cell according to [14].

[16] The method according to [15], further comprising the step of adding or expressing β-galactoside α-2,6-sialyltransferase 1 (ST6GAL1).

[17] An Fc region variant comprising the following amino acid variations (1) and (2):

(1)

(a) amino acid residues 234 and/or 235 are substituted by serine, threonine, asparagine, glutamine, cysteine, aspartic acid, glutamic acid, lysine, arginine, histidine, glycine, alanine, proline, methionine, valine, or isoleucine, wherein the amino acids at positions 234 and 235 are the same or different after substitution;
(b) amino acid residue 234 is substituted by phenylalanine, tyrosine, tryptophane, isoleucine, valine, methionine, proline, alanine, or glycine, and amino acid residue 235 is substituted by serine, threonine, asparagine, glutamine, cysteine, aspartic acid, glutamic acid, lysine, arginine, or histidine; or

(c) amino acid residue 234 is substituted by serine, threonine, asparagine, glutamine, cysteine, aspartic acid, glutamic acid, lysine, arginine, or histidine, and amino acid residue 235 is substituted by phenylalanine, tyrosine, tryptophane, isoleucine, valine, methionine, proline, alanine, or glycine; and

(2) amino acid residue 293 or 294 is deleted,
wherein the amino acid residue numbers are amino acid positions according to EU index.

[18] A pharmaceutical composition comprising the polypeptide according to any of [7] to [11] and a pharmaceutically acceptable carrier.
[19] The pharmaceutical composition according to [18] for use in the treatment of an autoimmune disease.
[20] The polypeptide according to any of [7] to [11] for use in the treatment of an autoimmune disease.
[21] A method for treating an autoimmune disease, comprising the step of administering a therapeutically effective amount of the polypeptide according to any of [7] to [11] to a subject.
[22] Use of the polypeptide according to any of [7] to [11] in the production of a pharmaceutical composition for the treatment of an autoimmune disease.

Advantageous Effects of Invention

The present invention can provide a sialylated Fc region variant with a further increased amount of sialic acid added.

Brief Description of Drawings

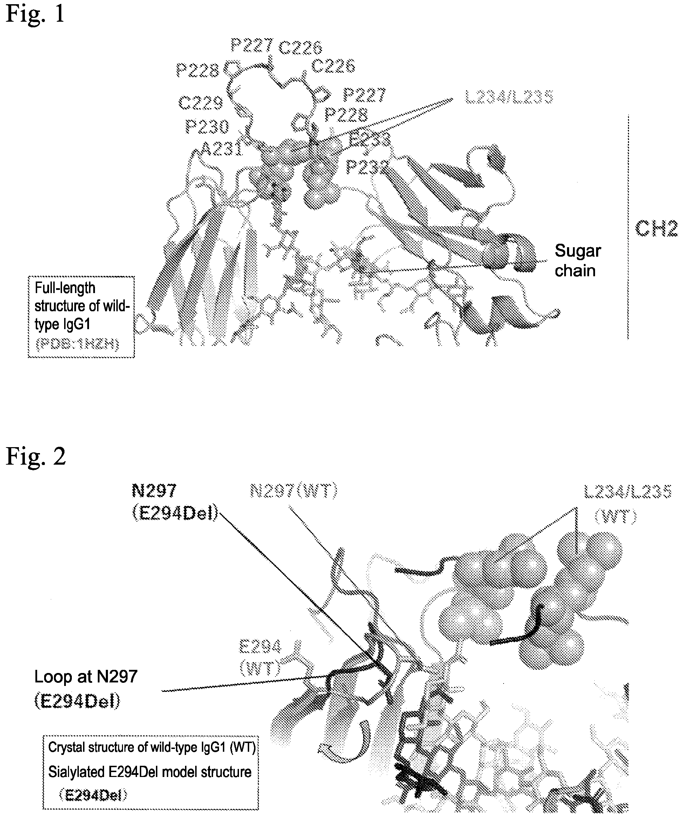

[Figure 1] Figure 1 is a diagram depicting a moiety from a hinge to a CH2 dimer in the full-length structure of IgG1 (PDB code: 1HZH). Leucine at positions 234 and 235 (L234/L235) is depicted by a plurality of spheres, and a sugar chain is depicted by a plurality of sticks.
[Figure 2] Figure 2 is a diagram depicting an N297 loop and its marginal structure by overlaying a sialylated E294Del mutant model structure with a wild-type structure. L234/L235 is depicted by a plurality of spheres, and a sugar chain is depicted by a plurality of sticks (gray: crystal structure of wild-type IgG1, black: sialylated E294Del mutant model structure). In the sialylated E294Del mutant, the N297 loop to be glycosylated is shortened, and the sugar chain is distorted, as compared with the wild type.

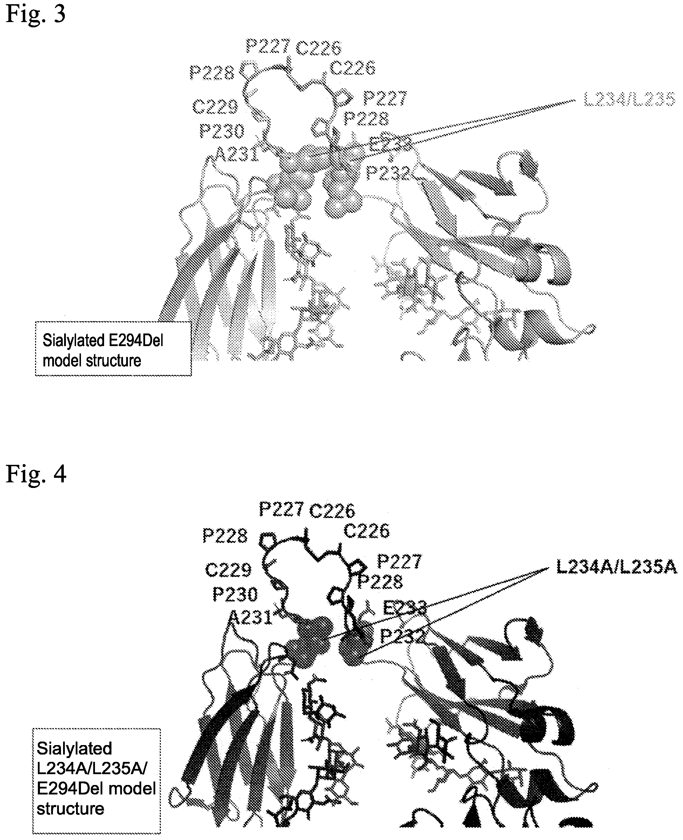

[Figure 3] Figure 3 is a diagram showing a sialylated E294Del mutant model structure and a marginal structure of L234/L235. L234/L235 is depicted by a plurality of spheres, and a sugar chain is depicted by a plurality of sticks.
[Figure 4] Figure 4 is a diagram showing a sialylated E294-deletion and L234A/L235A mutant model structure and a marginal structure of positions 234 and 235 (L234A/L235A). Alanine at positions 234 and 235 is depicted by a plurality of spheres, and a sugar chain is depicted by a plurality of sticks.

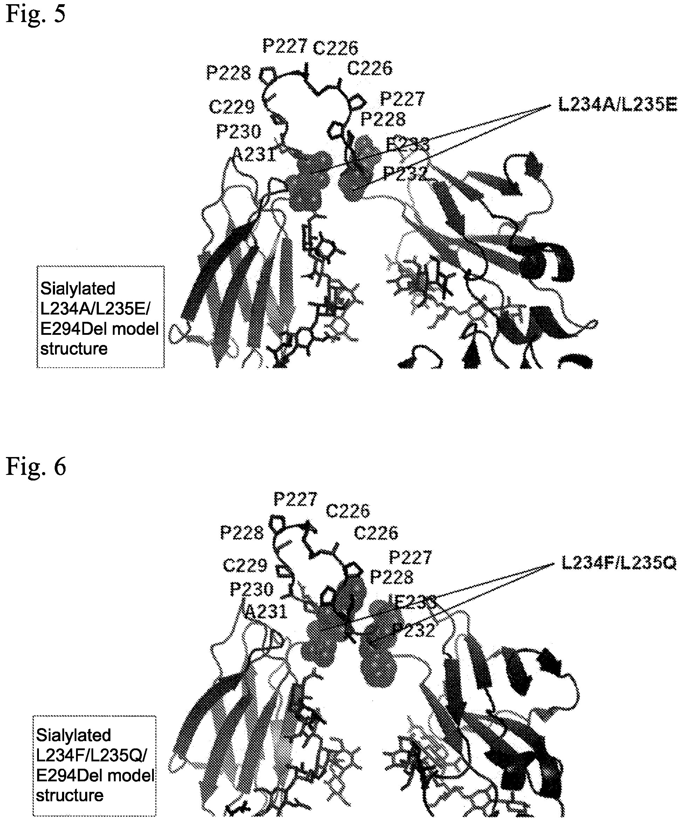

[Figure 5] Figure 5 is a diagram showing a sialylated E294-deletion and L234A/L235E mutant model structure and a marginal structure of positions 234 and 235 (L234A/L235E). Alanine at position 234 and glutamic acid at position 235 are each depicted by a plurality of spheres, and a sugar chain is depicted by a plurality of sticks.
[Figure 6] Figure 6 is a diagram showing a sialylated E294-deletion and L234F/L235Q mutant model structure and a marginal structure of positions 234 and 235 (L234F/L235Q). Phenylalanine at position 234 and glutamine at position 235 are each depicted by a plurality of spheres, and a sugar chain is depicted by a plurality of sticks.

Description of Embodiments

Hereinafter, the mode for carrying out the present invention will be described in detail. Materials, configurations, and the like described below do not limit the present invention, and various changes or modifications can be made in the present invention without departing from the spirit of the present invention.

1. Definition

Terms in the present specification are used in meanings generally used by those skilled in the art, unless otherwise specified.
The antibody (or immunoglobulin) refers to a glycoprotein having a four-chain structure having a bilaterally symmetrical Y-shaped structure composed of two heavy chains having a single sequence and two light chains having a single sequence. The antibody has five classes: IgG, IgM, IgA, IgD, and IgE. The basic structure of the antibody molecule is common among these classes, and two heavy chains having a molecular weight of 50,000 to 70,000 and two light chains having a molecular weight of 20,000 to 30,000 are bound through a disulfide bond and a noncovalent bond to form an antibody molecule having a Y-shaped four-chain structure with a molecular weight of 150,000 to 190,000. The heavy chain consists of a polypeptide chain usually containing approximately 440 amino acids, has a structure characteristic of each class, and is designated as Igγ, Igμ, Igα, Igδ, and Igε correspondingly to IgG, IgM, IgA, IgD, and IgE, respectively. IgG further has subclasses IgG1, IgG2, IgG3, and IgG4, and heavy chains corresponding thereto are designated as Igγ1, Igγ2, Igγ3, and Igγ4, respectively. The light chain consists of a polypeptide chain usually containing approximately 220 amino acids, and is known to have two types, L type and K type, which are designated as Igλ and Igκ, respectively. Each of these two types of light chains can be paired with any type of heavy chain.

In an antibody molecule, the heavy chain has four intrachain disulfide bonds (five in Igμ and Igε) while the light chain has two intrachain disulfide bonds, and one loop is formed every 100 to 110 amino acid residues. Their three-dimensional structures are similar among the loops and are designated as structural units or domains. In both the heavy chain and the light chain, a domain positioned at the N terminus is designated as a variable region, which has diverse amino acid sequences even among antibodies produced from the same class (or subclass) of the same species of animals, and is known to participate in antibody-antigen binding specific binding. A domain on the C-terminal side downstream of the variable region on the C-terminal side has an amino acid sequence substantially constant in each class or subclass, and is designated as a constant region. The heavy chain has, in the direction from the N terminus toward the C terminus, a heavy chain variable region (VH) and a heavy chain constant region (CH). CH is further divided, from the N-terminal side, into three domains: a CH1 domain, a CH2 domain, and a CH3 domain (in the case of IgG). The light chain has, in the direction from the N terminus toward the C terminus, a light chain variable region (VL) and a light chain constant region (CL).

The amino acid sequences of three complementarity-determining regions (CDRs) present in VH and VL vary very largely and contribute to the variability of the variable regions. CDRs reside in the order of CDR1, CDR2, and CDR3 at the N terminus of each of the heavy chain and the light chain, and these regions are composed of approximately 5 to 10 amino acid residues and form an antigen binding site. On the other hand, a portion except for CDRs of the variable region is designated as framework regions (FRs), which consist of FR1 to FR4 and comparatively little vary in amino acid sequence.

When an antibody is treated with protease papain, three antibody fragments are obtained. Two N-terminal fragments are designated as Fab (fragment, antigen binding) regions. As used herein, the term "Fab region" refers to a region composed of the heavy chain VH and CH1 domain and the light chain (VL and CL) and binds to an antigen through an antigen binding site at an end portion constituted by the Fab region. As used herein, the term "heavy chain fragment" refers to a fragment composed of the heavy chain VH and CH1 domain that constitute the Fab region.

The C-terminal fragment is designated as an Fc (fragment, crystallizable) region. As used herein, the "Fc region" means a polypeptide composed of an antibody constant region except for the CH1 domain. Thus, the Fc regions of IgA, IgD, and IgG have C-terminal last two heavy chain constant region domains (CH2 and CH3), and the Fc regions of IgE and IgM comprise C-terminal last three heavy chain constant region domains (CH2, CH3, and CH4). A hinge region, a J chain, and the like, if present, are also included in the term "Fc region". For example, the Fc region of human IgG1 includes a polypeptide composed of the CH2 domain and the CH3 domain, and a polypeptide composed of a portion or the whole of the hinge region, and the CH2 domain and the CH3 domain. Fc regions in antibodies of other classes can be determined by sequence alignment with the human IgG1 heavy chain or a fragment thereof.

As used herein, the term "Fc region variant" means an Fc region comprising at least one amino acid variation in a wild-type Fc region, and the amino acid variation includes amino acid substitution, addition, deletion, insertion, modification, and the like. As used herein, the "variation" and the "mutation" of an amino acid are used interchangeably with each other. As used herein, the term "sialylated Fc region variant" means an Fc region variant sialylated (i.e., having a sialic acid added thereto) by a method known in the art. As used herein, the term "Fc region variant" is used as a term including both of a non-sialylated Fc region variant and the sialylated Fc region variant, unless particularly limited by the context. As used herein, the term "Fc region" or "Fc region variant" used is meant to also include a complex (e.g., a dimer) composed of a plurality of Fc regions.

A one-armed antibody and a one-armed Fc fusion protein are also included in the scope of the present invention. For example, a one-armed IgG antibody comprises a complex of one Fab region and two Fc regions and has a structure where the heavy chain fragment of the Fab region is linked to one of the two Fc regions. In one embodiment, the one-armed antibody comprises one heavy chain (VH, CH1 domain, and sialylated Fc region variant (hinge region + CH2 domain + CH3 domain)), one light chain (VL and CL), and a sialylated Fc region variant. In one embodiment, the one-armed Fc fusion protein has a structure where only one of two sialylated Fc region variants in a complex is bound to another protein or peptide.

As used herein, the term "multispecific antibody" refers to an antibody that can specifically bind to two or more different antigens, and is designated as, for example, a bispecific antibody or a trispecific antibody, depending on the number of antigens to be bound. The multispecific antibody comprises a complex of two or more antibodies and/or antigen binding fragments that can bind to different antigens, respectively. The "antibody" as used herein includes the multispecific antibody, unless particularly limited by the context.

As used herein, the term "bispecific antibody" refers to an antibody that can specifically bind to two different antigens.

As used herein, the term "human antibody" refers to an antibody having the amino acid sequence of a human immunoglobulin. As used herein, the term "humanized antibody" refers to an antibody having amino acid residues, except for CDR, substituted partially, mostly, or wholly by amino acid residues derived from a human immunoglobulin molecule. The humanization method is not particularly limited, and the humanized antibody can be prepared with reference to, for example, U.S. Patent Nos. 5225539 and 6180370.

The amino acid residue numbers of an Fc region or an antibody used in the present specification are prescribed in accordance with the EU index of Kabat et al. (Kabat et al., Sequences of Proteins of Immunological Interest, 5th Ed, 1991, NIH Publication No. 91-3242).

As used herein, the term "linkage" or "linked" means that a plurality of components (e.g., a Fab region and an Fc region) are bound directly or via one or more mediators (e.g., peptide linkers). As used herein, the "peptide linker" means one or more arbitrary amino acid residues that can be introduced by genetic engineering in order to link two or more moieties (e.g., between an antibody heavy chain variable region and light chain variable region). The length of the peptide linker used in the present invention is not particularly limited and can be appropriately selected by those skilled in the art depending on a purpose.

As used herein, the term "identity" means an identity value obtained with parameters provided as defaults using EMBOSS Needle (Nucleic Acids Res., 2015, Vol. 43, p W580-W584). The parameters are as follows.

Gap Open Penalty = 10
Gap Extend Penalty = 0.5
Matrix = EBLOSUM62
End Gap Penalty = false

As used herein, the term "subject" means a human or other animals in need of prevention or treatment. In a certain aspect, the subject is a human in need of prevention or treatment.

2. Sialylated Fc region variant

The sialylated Fc region variant according to one embodiment of the present invention comprises the following amino acid variations (1) and (2):

(1)

(a) amino acid residues 234 and/or 235 are substituted by serine, threonine, asparagine, glutamine, cysteine, aspartic acid, glutamic acid, lysine, arginine, histidine, glycine, alanine, proline, methionine, valine, or isoleucine (wherein the amino acids at positions 234 and 235 are the same or different after substitution);
(b) amino acid residue 234 is substituted by phenylalanine, tyrosine, tryptophane, isoleucine, valine, methionine, proline, alanine, or glycine, and amino acid residue 235 is substituted by serine, threonine, asparagine, glutamine, cysteine, aspartic acid, glutamic acid, lysine, arginine, or histidine; or
(c) amino acid residue 234 is substituted by serine, threonine, asparagine, glutamine, cysteine, aspartic acid, glutamic acid, lysine, arginine, or histidine, and amino acid residue 235 is substituted by phenylalanine, tyrosine, tryptophane, isoleucine, valine, methionine, proline, alanine, or glycine; and

(2) amino acid residue 293 or 294 is deleted
(wherein the amino acid residue numbers are amino acid positions according to the EU index).

Specifically, the sialylated Fc region variant according to one embodiment of the present invention comprises both the amino acid variations (1) and (2) and is required to comprise one of the amino acid variations (a) to (c) for (1). The sialylated Fc region variant has an increased amount of sialic acid added (i.e., degree of sialylation) as compared with sialylated wild-type Fc region or a sialylated Fc region variant having only the deletion of an amino acid at position 294. The sialylated Fc region variant according to an embodiment particularly has an increased amount of sialic acid added to the sugar chain linked to asparagine at position 297.

The reason why the amino acid variations as mentioned above increase the amount of sialic acid added (particularly, sialic acid added to the sugar chain linked to asparagine at position 297) is presumably as described below.

Although an amino acid variation (particularly, deletion) at position 294 of an Fc region is known to be capable of contributing to increase in sialic acid addition (Patent Literature 2), the present inventors have found that the deletion of the amino acid at position 294 may cause a distortion at a sugar chain start position (around position 297). Figure 1 is a diagram showing the three-dimensional structure of a wild-type IgG CH2 domain. Figure 2 is a diagram showing the three-dimensional structure of a form in which glutamic acid at position 294 of wild-type IgG is deleted. As shown in Figure 1, in the wild-type IgG, a CH2 dimer interface is composed mainly of a sugar chain and leucine at positions 234 and 235. The deletion of glutamic acid at position 294 shortens a loop including asparagine at position 297 serving as a sugar chain starting position, as shown in Figure 2, resulting in displacement around the sugar chain start position so that the whole sugar chain is distorted.

Figure 3 is a diagram showing the three-dimensional structure of a sugar chain distortion caused by the deletion of glutamic acid at position 294. As a result of such a sugar chain distortion, the interaction between leucine at position 234 and leucine at position 235 is mainly involved in CH2 dimer contact. Leucine at position 234 and leucine at position 235 are bound to each other through Van der Waals interaction and therefore have no space therebetween, and a further distortion in the sugar chain is thus not accepted.

The present inventors have introduced variations to the amino acids at positions 234 and/or 235 so as to decrease the interaction therebetween, and consequently found that the amount of sialic acid added to the sugar chain is increased. This is presumably because when the decreased interaction between positions 234 and 235 causes three-dimensional room such as space between these sites, and a further distortion in the sugar chain is thereby accepted. The sugar chain is distorted to a position that facilitates sialic acid addition, and the amount of sialic acid added to the sugar chain is thereby increased. Figure 4 is a diagram showing the three-dimensional structure of a form in which amino acid residue 294 is deleted and in addition, each leucine at positions 234 and 235 is substituted by alanine relative to wild-type IgG. According to Figure 4, when each leucine at positions 234 and 235 is substituted by alanine, the Van der Waals interaction therebetween is weakened, resulting in space between these sites. Since there can be room for the molecular structure, a further distortion in the sugar chain is accepted. As a result, the amount of sialic acid added is presumably also increased.

The amino acid substitutions at positions 234 and/or 235 are not limited to the forms mentioned above, and desired effects can be obtained as long as the amino acids at the positions are selected so as to decrease the interaction between the amino acids at positions 234 and 235. Possible variations that decrease the interaction between the amino acids at positions 234 and 235 are variations that weaken the Van der Waals interaction between the amino acids at positions 234 and 235. The variations that weaken the Van der Waals interaction between the amino acids at positions 234 and 235 presumably include, for example, the substitution of at least one of the amino acids at positions 234 and 235 by a less hydrophobic or more hydrophilic amino acid than the original amino acid and/or a smaller (less bulky) amino acid than the original amino acid. For example, when both the amino acids at positions 234 and 235 are leucine before substitution, one or both of these amino acids may be substituted by a less hydrophobic amino acid than leucine, i.e., serine, threonine, asparagine, glutamine, cysteine, aspartic acid, glutamic acid, lysine, arginine, histidine, glycine, alanine, proline, or methionine (J Mol Biol., 1982, Vol. 157 (1), p. 105-32). Amino acids are arranged in order of bulkiness as follows: tryptophane > tyrosine > arginine > phenylalanine > histidine > methionine > glutamic acid > lysine > glutamine > aspartic acid > asparagine > leucine > isoleucine > cysteine > threonine > valine > proline > serine > alanine > glycine. Accordingly, the substitution of at least one of the amino acids at positions 234 and 235 by a smaller (less bulky) amino acid than the original amino acid means that each amino acid is substituted by a less bulky amino acid than the amino acid before substitution, with reference to the order of bulkiness mentioned above. For example, when both the amino acids at positions 234 and 235 are leucine before substitution, one or both of these amino acids may be substituted by a less bulky amino acid, i.e., isoleucine, cysteine, threonine, valine, proline, serine, alanine, or glycine. Alternatively, when one of the amino acids at positions 234 and 235 is substituted by a hydrophilic amino acid (serine, threonine, asparagine, glutamine, cysteine, aspartic acid, glutamic acid, lysine, arginine, or histidine), the other amino acid may be substituted by a more bulky hydrophobic amino acid than the amino acid before substitution. For example, when both the amino acids at positions 234 and 235 are leucine before substitution, one of these amino acids may be substituted by a hydrophilic amino acid and the other amino acid may be substituted by a more bulky hydrophobic amino acid tryptophane, tyrosine, phenylalanine, or methionine. Examples of such an amino acid variation that decreases the interaction between the amino acids at positions 234 and 235 include the amino acid variation (1) described in the above embodiment.

Examples of the combination of the variations that weaken the Van der Waals interaction between the amino acids at positions 234 and 235 include, when any one of the amino acid residues 234 and 235 is leucine, the substitution of the other amino acid residue by serine, threonine, asparagine, glutamine, cysteine, aspartic acid, glutamic acid, lysine, arginine, histidine, glycine, alanine, proline, methionine, valine, or isoleucine.

Examples of the combination of the amino acids at positions 234 and 235 that weaken the Van der Waals interaction also include the following combinations:

(1) isoleucine-lysine, isoleucine-arginine, isoleucine-asparagine, isoleucine-aspartic acid, isoleucine-glutamine,

EP 4 772 534 A1

isoleucine-glutamic acid, isoleucine-histidine, isoleucine-serine, isoleucine-threonine, isoleucine-methionine, isoleucine-glycine, isoleucine-alanine, isoleucine-cysteine, isoleucine-proline, isoleucine-valine, or isoleucine-isoleucine; and

(2) valine-lysine, valine-arginine, valine-asparagine, valine-aspartic acid, valine-glutamine, valine-glutamic acid, valine-histidine, valine-serine, valine-threonine, valine-methionine, valine-glycine, valine-alanine, valine-cysteine, valine-proline, valine-valine, or valine-isoleucine;

(3) methionine-lysine, methionine-arginine, methionine-asparagine, methionine-aspartic acid, methionine-glutamine, methionine-glutamic acid, methionine-histidine, methionine-serine, methionine-threonine, methionine-methionine, methionine-glycine, methionine-alanine, methionine-cysteine, methionine-proline, methionine-valine, or methionine-isoleucine;

(4) proline-lysine, proline-arginine, proline-asparagine, proline-aspartic acid, proline-glutamine, proline-glutamic acid, proline-histidine, proline-serine, proline-threonine, proline-methionine, proline-glycine, proline-alanine, proline-cysteine, proline-proline, proline-valine, or proline-isoleucine;

(5) cysteine-lysine, cysteine-arginine, cysteine-asparagine, cysteine-aspartic acid, cysteine-glutamine, cysteine-glutamic acid, cysteine-histidine, cysteine-serine, cysteine-threonine, cysteine-methionine, cysteine-glycine, cysteine-alanine, cysteine-proline, cysteine-valine, or cysteine-isoleucine;

(6) alanine-lysine, alanine-arginine, alanine-asparagine, alanine-aspartic acid, alanine-glutamine, alanine-glutamic acid, alanine-histidine, alanine-serine, alanine-threonine, alanine-methionine, alanine-glycine, alanine-alanine, alanine-cysteine, alanine-proline, alanine-valine, or alanine-isoleucine;

(7) glycine-lysine, glycine-arginine, glycine-asparagine, glycine-aspartic acid, glycine-glutamine, glycine-glutamic acid, glycine-histidine, glycine-serine, glycine-threonine, glycine-methionine, glycine-glycine, glycine-alanine, glycine-cysteine, glycine-proline, glycine-valine, or glycine-isoleucine;

(8) threonine-lysine, threonine-arginine, threonine-asparagine, threonine-aspartic acid, threonine-glutamine, threonine-glutamic acid, threonine-histidine, threonine-serine, threonine-threonine, threonine-methionine, threonine-glycine, threonine-alanine, threonine-cysteine, threonine-proline, threonine-valine, or threonine-isoleucine;

(9) serine-lysine, serine-arginine, serine-asparagine, serine-aspartic acid, serine-glutamine, serine-glutamic acid, serine-histidine, serine-serine, serine-threonine, serine-methionine, serine-glycine, serine-alanine, serine-cysteine, serine-proline, serine-valine, or serine-isoleucine;

(10) lysine-lysine, lysine-arginine, lysine-asparagine, lysine-aspartic acid, lysine-glutamine, lysine-glutamic acid, lysine-histidine, lysine-serine, lysine-threonine, lysine-methionine, lysine-glycine, lysine-alanine, lysine-cysteine, lysine-proline, lysine-valine, or lysine-isoleucine;

(11) histidine-lysine, histidine-arginine, histidine-asparagine, histidine-aspartic acid, histidine-glutamine, histidine-glutamic acid, histidine-histidine, histidine-serine, histidine-threonine, histidine-methionine, histidine-glycine, histidine-alanine, histidine-cysteine, histidine-proline, histidine-valine, or histidine-isoleucine;

(12) glutamine-lysine, glutamine-arginine, glutamine-asparagine, glutamine-aspartic acid, glutamine-glutamine, glutamine-glutamic acid, glutamine-histidine, glutamine-serine, glutamine-threonine, glutamine-methionine, glutamine-glycine, glutamine-alanine, glutamine-cysteine, glutamine-proline, glutamine-valine, or glutamine-isoleucine;

(13) glutamic acid-lysine, glutamic acid-arginine, glutamic acid-asparagine, glutamic acid-aspartic acid, glutamic acid-glutamine, glutamic acid-glutamic acid, glutamic acid-histidine, glutamic acid-serine, glutamic acid-threonine, glutamic acid-methionine, glutamic acid-glycine, glutamic acid-alanine, glutamic acid-cysteine, glutamic acid-proline, glutamic acid-valine, or glutamic acid-isoleucine;

(14) asparagine-lysine, asparagine-arginine, asparagine-asparagine, asparagine-aspartic acid, asparagine-glutamine, asparagine-glutamic acid, asparagine-histidine, asparagine-serine, asparagine-threonine, asparagine-methionine, asparagine-glycine, asparagine-alanine, asparagine-cysteine, asparagine-proline, asparagine-valine, or asparagine-isoleucine;

(15) aspartic acid-lysine, aspartic acid-arginine, aspartic acid-asparagine, aspartic acid-aspartic acid, aspartic acid-glutamine, aspartic acid-glutamic acid, aspartic acid-histidine, aspartic acid-serine, aspartic acid-threonine, aspartic acid-methionine, aspartic acid-glycine, aspartic acid-alanine, aspartic acid-cysteine, aspartic acid-proline, aspartic acid-valine, or aspartic acid-isoleucine;

(16) arginine-lysine, arginine-arginine, arginine-asparagine, arginine-aspartic acid, arginine-glutamine, arginine-glutamic acid, arginine-histidine, arginine-serine, arginine-threonine, arginine-methionine, arginine-glycine, arginine-alanine, arginine-cysteine, arginine-proline, arginine-valine, or arginine-isoleucine;

(17) tryptophane-lysine, tryptophane-arginine, tryptophane-asparagine, tryptophane-aspartic acid, tryptophane-glutamine, tryptophane-glutamic acid, tryptophane-histidine, tryptophane-serine, tryptophane-threonine, or tryptophane-cysteine;

(18) tyrosine-lysine, tyrosine-arginine, tyrosine-asparagine, tyrosine-aspartic acid, tyrosine-glutamine, tyrosine-glutamic acid, tyrosine-histidine, tyrosine-serine, tyrosine-threonine, or tyrosine-cysteine; and

(19) phenylalanine-lysine, phenylalanine-arginine, phenylalanine-asparagine, phenylalanine-aspartic acid, phenylalanine-glutamine, phenylalanine-glutamic acid, phenylalanine-histidine, phenylalanine-serine, phenylalanine-threonine, or phenylalanine-cysteine

(wherein in the combination "amino acid I-amino acid II", the amino acid residue 234 may be the amino acid I, and the amino acid residue 235 may be the amino acid II, or vice versa).

Preferred specific examples thereof include the substitution of leucine at positions 234 and 235 by alanine. Other preferred specific examples thereof include the substitution of leucine at position 234 by alanine and leucine at position 235 by glutamic acid. In this case, results of analyzing a model structure are shown in Figure 5. Figure 5 is a diagram showing the three-dimensional structure of a form in which amino acid residue 294 is deleted and in addition, leucine at position 234 is substituted by alanine, and leucine at position 235 is substituted by glutamic acid relative to wild-type IgG. According to Figure 5, the respective side chains of alanine at position 234 and glutamic acid at position 235 face each other, and the Van der Waals interaction therebetween is weakened, resulting in space between these sites. Since there can be room for the molecular structure, a further distortion in the sugar chain is accepted. As a result, the amount of sialic acid added is presumably also increased.

Other preferred specific examples thereof may include the substitution of leucine at position 234 by phenylalanine and leucine at position 235 by glutamine. In this case, results of analyzing a structure are shown in Figure 6. Figure 6 is a schematic view of a form in which amino acid residue 294 is deleted and in addition, leucine at position 234 is substituted by phenylalanine, and leucine at position 235 is substituted by glutamine relative to wild-type IgG. According to Figure 6, the respective side chains of phenylalanine at position 234 and glutamine at position 235 face each other, and the Van der Waals interaction therebetween is weakened, resulting in space between these sites. Since there can be room for the molecular structure, a further distortion in the sugar chain is accepted. As a result, the amount of sialic acid added is presumably also increased.

In the structural analysis of Figures 1 to 6, modeling was performed with WinCoot (Coot 0.9.6 Marina Bay), the drawings were prepared with PyMOL (open source Version 2.60a0), and the interaction was evaluated with Schrodinger Maestro (Maestro Version 12.4.072).

The sialylated Fc region variant according to an embodiment has an increased amount of sialic acid added and therefore exhibits excellent pharmacokinetics such as an increased half-life and exerts an excellent anti-inflammatory effect. In other words, the sialylated Fc region variant also has the advantage of a low dose when used as a medicament, because of its high therapeutic effects.

A problem of frequent occurrence with antibody drugs is *in vivo* production of an anti-drug antibody (ADA) upon administration. However, the sialylated Fc region variant according to an embodiment is unlikely to cause *in vivo* production of an anti-drug antibody (ADA) after administration. This means that a medicament containing the sialylated Fc region variant according to an embodiment, even when repeatedly used, persistently produces desired effects, leading to very large advantages.

Furthermore, the deletion of amino acids at positions 293 and/or 294 can attenuate the binding activity of the antibody drug used against FcγRI and as a result, reduces adverse reactions. This effect is potentiated by the substitution of the amino acids at positions 234 and/or 235 as mentioned above.

As mentioned above, the amino acid variations at positions 234 and/or 235 are not limited as long as the amino acids at the positions are selected so as to decrease the interaction between the amino acids at positions 234 and 235.

In a preferred embodiment, amino acid residues 234 and 235 are substituted by serine, threonine, asparagine, glutamine, cysteine, aspartic acid, glutamic acid, lysine, arginine, histidine, glycine, alanine, proline, methionine, valine, or isoleucine (wherein the amino acids at positions 234 and 235 are the same or different after substitution).

In a more preferred embodiment, amino acid residues 234 and 235 are substituted by alanine, or amino acid residue 234 is substituted by alanine, amino acid residue 235 is substituted by glutamic acid.

In the sialylated Fc region variant of the present invention, an amino acid at position 293 or 294 is deleted. Since both the amino acids at positions 293 and 294 are usually glutamic acid in wild-type IgG, the deletion of either of them produces the same variation. In a preferred embodiment, glutamic acid at position 293 or 294 is deleted.

The origin of the Fc region is not particularly limited. For example, an Fc region of IgA1, IgA2, IgD, IgE, IgG1, IgG2, IgG3, IgG4, or IgM type exists as an antibody Fc region. The Fc region variant according to an embodiment may be derived from any of these antibodies and is preferably derived from IgG (IgG1, IgG2, IgG3, or IgG4), more preferably from human IgG, further preferably from human Igγ1, human Igγ2, human Igγ3, or human Igγ4, particularly preferably from human Igγ1.

Particularly, amino acid mutations L234A and L235A in a human Igγl constant region are called "LALA mutations". In this context, L234A is the substitution of leucine at position 234 according to the EU index in the human Igγ1 constant region by alanine. L235A is the substitution of leucine at position 235 according to the EU index in the human Igγ1 constant region by alanine. These mutations are known to reduce the antibody-dependent cellular cytotoxicity or complement-dependent cytotoxicity of an antibody (Mol. Immunol., 1992, Vol. 29, p. 633-639).

The sialylated Fc region variant according to an embodiment has an increased amount of sialic acid added (i.e., degree of sialylation) as compared with sialylated wild-type Fc region or a sialylated Fc region variant having only the deletion of an amino acid at position 294. The Fc region variant according to an embodiment particularly has an increased amount of sialic acid added to the sugar chain linked to asparagine at position 297, though the position of sialic acid addition is not limited to the position. The amount of sialic acid added can be measured as described in Examples mentioned later, and indicated as a "sialylation rate (%)". As used herein, the "sialylation rate (%)" means the ratio of a sialic acid-containing sugar chain to N-linked sugar chains present in a material to be measured, and a specific measurement method and calculation method are as described in Examples mentioned later.

The sialylation rate of the sialylated Fc region variant according to an embodiment is increased by, for example, 2 to 10%, 3 to 8%, or 3 to 6%, as compared with a variant having only the deletion of an amino acid at position 294.

The sialylated Fc region variant according to an embodiment can be obtained by a method usually used in the art, and the amino acid variations and the sialic acid addition can also be performed by methods usually used in the art. A method of producing a sialylated Fc region variant by allowing cells to co-express plasmids respectively encoding β galactosyltransferase and sialyltransferase with a plasmid encoding an Fc region variant is known as a method for increasing the amount of sialic acid added in an Fc region, though not limited thereto.

The sialylated Fc region variant according to an embodiment may comprise a variation other than those mentioned above. Although such a variation is not particularly limited as long as a desired sialylation rate can be achieved, the sialylated Fc region variant may comprise, for example, a S239D/I332E variation (Proc. Natl. Acad. Sci. USA, 2006, Vol. 103 (11), p. 4005-4010) and variations based on the knobs-into-holes technology (hereinafter, also referred to as "knobs-into-holes variations"). The knobs-into-holes technology is a technique that can efficiently obtain a heterodimeric antibody molecule of interest by replacing amino acid side chains present in a CH3 region in one of the heavy chains with larger side chains (knobs), replacing amino acid side chains present in a CH3 region of the other heavy chain with smaller side chains (holes), and placing the knobs into the holes to promote the heterodimerization of the heavy chains (Nature Biotech., 1998, Vol. 16, p. 677-681; J. Mol. Biol., 1997, Vol. 270, p. 26-35; and Proc. Natl. Acad. Sci. USA, 2013, Vol. 110, p. E2987-E2996).

Specifically, the sialylated Fc region variant according to an embodiment can have an Fc region moiety in the amino acid sequence of a heavy chain represented by SEQ ID NO: 7 or 13 described in Examples, i.e., a sequence consisting of amino acids at positions 218 to 448 in the amino acid sequence represented by SEQ ID NO: 7 or a sequence consisting of amino acids at positions 217 to 447 in the amino acid sequence represented by SEQ ID NO: 13. The Fc region variant according to an embodiment may comprise a moiety different from these sequences as long as desired effects are exerted. The Fc region variant according to an embodiment has, for example, an amino acid sequence 90% or more, preferably 95% or more, identical to the Fc region moiety in the amino acid sequence of a heavy chain represented by SEQ ID NO: 7 or 13.

3. Polypeptide comprising sialylated Fc region variant

According to one embodiment, the present invention provides a polypeptide comprising the sialylated Fc region variant mentioned above. The type of the polypeptide is not limited as long as the polypeptide comprises the sialylated Fc region variant mentioned above. Examples thereof include antibodies. Accordingly, according to one embodiment, the present invention provides an antibody comprising the sialylated Fc region variant mentioned above.

As used herein, the term "antibody" is used in the broadest sense and includes every antibody such as a monoclonal antibody (including a full-length monoclonal antibody), a polyclonal antibody, an antibody mutant, an antibody fragment, a multispecific antibody (e.g., a bispecific antibody), a chimeric antibody, and a humanized antibody, as long as the antibody exhibits desired biological activity. The antibody may be a one-armed antibody. The antibody according to an embodiment may be an antibody of any of classes IgG, IgM, IgA, IgD, and IgE and is, for example, an IgG antibody.

The antibody according to an embodiment is not limited by the type of an antigen, the origin of the antibody, or the like and may be any antibody. Examples of the origin of the antibody can include, but are not particularly limited to, human antibodies, mouse antibodies, rat antibodies, and rabbit antibodies.

Moieties (light chain and heavy chain variable regions, etc.) other than the Fc region in the antibody according to an embodiment can have arbitrary sequences and may have sequences of an antibody of any origin, such as a mouse antibody, a rat antibody, a rabbit antibody, a goat antibody, a camel antibody, a humanized antibody obtained by the humanization of such a non-human antibody, and a human antibody. Variations at amino acid residues are also accepted as long as the antigen binding activity is maintained. In the case of introducing variations to the amino acid sequence of a variable region, a variation site and the number of amino acids that receives variations are not particularly limited.

The antibody may have a peptide linker that links its two or more moieties (e.g., the heavy chain variable region and the light chain variable region of the antibody). Its type and length are not particularly limited and may be appropriately selected by those skilled in the art. The length is preferably 5 or more amino acids (the upper limit of which is not particularly limited and is usually 30 or less amino acids, preferably 20 or less amino acids), particularly preferably 15 amino acids. For example, a glycine-serine linker (GS linker) or a glycine-lysine-proline-glycine-serine linker (GKPGS linker) can be used as the peptide linker that links the heavy chain variable region and the light chain variable region of the antibody.

The antibody according to an embodiment can be produced in accordance with a method known in the art. A specific method is as described in "6. Method for producing polypeptide" mentioned later.

The polypeptide comprising the sialylated Fc region variant according to an embodiment may be an Fc fusion protein molecule of the sialylated Fc region variant linked to an additional protein, a bioactive peptide, or the like. Examples of the additional protein and the bioactive peptide include, but are not limited to, receptors, adhesion molecules, ligands, and enzymes.

Preferred examples of the Fc fusion protein molecule include protein molecules of the Fc region (the sialylated Fc region variant according to an embodiment) fused with a receptor protein that binds to a target. Examples of such a protein molecule include TNFR-Fc fusion proteins, IL1R-Fc fusion proteins, and VEGFR-Fc fusion proteins. Further examples of the protein or the peptide to be fused include scFv molecules, single-domain antibody molecules, and antibody-like molecules (e.g., DARPins, Affibody, Avimer, and Adnectin). The Fc fusion protein molecule may have multispecificity that permits binding to plural types of target molecules or epitopes. When a plurality of Fc region variants form a complex, the additional protein or peptide may be linked to only one or some of the Fc region variants. For example, when two Fc region variants form a complex, the additional protein or peptide may be linked to only one of the Fc region variants to form a one-armed Fc fusion protein.

The Fc fusion protein molecule may comprise a non-human derived protein. The Fc fusion protein can be repetitively administered to the same subject because the non-human derived protein linked to the sialylated Fc region suppresses ADA production.

The Fc fusion protein molecule according to an embodiment can be produced in accordance with a method known in the art. A specific method is as described in "6. Method for producing polypeptide" mentioned later.

4. Polynucleotide and expression vector

According to one embodiment, the present invention provides a polynucleotide encoding the Fc region variant of the present invention, and a polynucleotide encoding the polypeptide comprising the sialylated Fc region variant mentioned above. Those skilled in the art are capable of preparing the polynucleotide according to an embodiment by use of a method known in the art on the basis of its nucleotide sequence. The polynucleotide according to an embodiment may be synthesized by use of, for example, a gene synthesis method known in the art. Various methods known to those skilled in the art, such as an antibody gene synthesis method described in International Publication No. WO 90/07861, may be used as such gene synthesis methods.

According to a further embodiment, the present invention provides an expression vector comprising the polynucleotide mentioned above. A polynucleotide may be separately contained in different vectors, or a plurality of polynucleotides may be contained in one vector.

The expression vector according to an embodiment is not particularly limited as long as the polynucleotide according to an embodiment can be produced in various host cells such as eukaryotic cells (e.g., animal cells, insect cells, plant cells, or yeasts) or prokaryotic cells (e.g., *E. coli*). Examples of such an expression vector include plasmid vectors and virus vectors. For example, pcDNA series (Thermo Fisher Scientific Inc.), pALTER(R)-MAX (Promega Corp.), pHEK293 Ultra Expression Vector (Takara Bio Inc.), or pEE6.4 or pEE12.4 (Lonza Biologics plc) can be used as the plasmid vector. For example, lentivirus, adenovirus, retrovirus, or adeno-associated virus can be used as the virus vector. For example, in the case of using lentivirus for introducing the polynucleotide according to an embodiment to cells, pLVSIN-CMV/EF1$\alpha$ vector (Takara Bio Inc.), pLenti vector (Thermo Fisher Scientific Inc.), or the like can be used as the lentivirus. In one embodiment, the vector for use in the expression vector according to an embodiment is pcDNA(TM)3.4-TOPO(R) vector (Thermo Fisher Scientific Inc.) and pcDNA(TM)3.1 vector (Thermo Fisher Scientific Inc.).

The expression vector according to an embodiment may contain a promoter operably linked to the polynucleotide of the present invention. Examples of the promoter for expressing the polynucleotide according to an embodiment in animal cells include promoters derived from viruses such as CMV, RSV, and SV40, actin promoter, EF (elongation factor) 1$\alpha$ promoter, and heat shock promoter. Examples of the promoter for expressing the polynucleotide according to an embodiment in a bacterium (e.g., a bacterium of the genus *Escherichia*) include trp promoter, lac promoter, $\lambda$PL promoter, and tac promoter. Examples of the promoter for expressing the polynucleotide according to an embodiment in a yeast include GAL1 promoter, GAL10 promoter, PH05 promoter, PGK promoter, GAP promoter, and ADH promoter.

In the case of using animal cells, insect cells, or a yeast as host cells, the expression vector according to an embodiment may contain a start codon and a stop codon. In this case, the expression vector may contain, for example, an enhancer sequence, 5' and 3' untranslated regions of a gene encoding the Fc region variant according to an embodiment, a secretory signal sequence, a splicing junction, a polyadenylation site, or a replicable unit. In the case of using *E. coli* as host cells, the expression vector according to an embodiment may contain a start codon, a stop codon, a terminator region, and a replicable unit. The expression vector according to an embodiment may contain a drug selection marker gene (e.g., tetracycline resistance gene, ampicillin resistance gene, kanamycin resistance gene, neomycin resistance gene, or dihydrofolate reductase gene) usually used depending on a purpose.

5. Host cell

According to one embodiment, the present invention provides a host cell transformed with the expression vector mentioned above. The host cell according to an embodiment may contain one or more polynucleotides according to an embodiment by transformation with the expression vector according to an embodiment.

The host cell to be transformed is not particularly limited as long as the host cell is compatible with the expression vector used and can express the polypeptide according to an embodiment by transformation with the expression vector. Various cells such as conventional cells usually used in the art or artificially established cells can be used as the host cell to be transformed. Examples thereof include animal cells (CHO-K1 cells, ExpiCHO-S(TM) cells, CHOK1SV cells, CHO-DG44 cells, HEK293 cells, NS0 cells, etc.), insect cells (Sf9, etc.), bacteria (bacteria of the genus *Escherichia,* etc.), and yeasts (the genus *Saccharomyces,* the genus *Pichia,* etc.). In one embodiment, the host cell is CHO-K1 cells or ExpiCHO-S cells.

A method for transforming the host cell is not particularly limited. For example, a method generally used by those skilled in the art, such as a calcium phosphate method, electroporation, or lipofection can be used.

The transformed host cell can be selected by a method generally used by those skilled in the art. For example, a cell isolation method such as a drug selection method using a drug selection marker gene and a drug such as tetracycline, ampicillin, neomycin, or hygromycin, a limiting dilution method, a single cell sorting method, or a colony pickup method can be used as the selection method.

6. Method for producing polypeptide

According to one embodiment, the present invention provides a method for producing the sialylated Fc region variant and a method for producing the polypeptide comprising the sialylated Fc region variant mentioned above. The method according to an embodiment may comprise, for example, the steps of: culturing the transformed host cell mentioned above so that the polypeptide is expressed into the host cell or a culture supernatant; and recovering, isolating, or purifying the polypeptide, though not limited to these methods as long as the polypeptide of interest is produced.

The transformed host cell can be cultured by a method known in the art. Culture conditions, for example, temperature, pH of a medium, and a culture time, are appropriately selected by those skilled in the art. When the host cell is animal cells, for example, MEM medium (Science, 1959, Vol. 130, p. 432-437), DMEM medium (Virol., 1959, Vol. 8, p. 396), RPMI-1640 medium (J. Am. Med. Assoc., 1967, Vol. 199, p. 519), or 199 medium (Exp. Biol. Med., 1950, Vol. 73, p. 1-8) containing approximately 5 to 20% fetal bovine serum can be used as the medium. The pH of the medium is, for example, approximately 6 to 8. The culture is usually performed at approximately 30 to 40°C for approximately 15 to 336 hours, if necessary, with aeration or stirring. When the host cell is insect cells, for example, Grace's medium (Proc. Natl. Acad. Sci. USA., 1985, Vol. 82, p. 8404) containing fetal bovine serum can be used as the medium. The pH of the medium is, for example, approximately 5 to 8. The culture is usually performed at approximately 20 to 40°C for approximately 15 to 100 hours, if necessary, with aeration or stirring.

When the host cell is *E. coli* or a yeast, for example, a liquid medium containing a nutrient is appropriate as the medium. The nutritive medium contains, for example, a carbon source and an inorganic nitrogen source or an organic nitrogen source necessary for the growth of the transformed host cell. Examples of the carbon source include glucose, dextran, soluble starch, and sucrose. Examples of the inorganic nitrogen source or the organic nitrogen source include ammonium salts, nitrates, amino acids, corn steep liquor, peptone, casein, meat extracts, soybean cake, and potato extracts. Other nutrients (e.g., inorganic salts (e.g., calcium chloride, sodium dihydrogen phosphate, and magnesium chloride) and vitamins) and antibiotics (e.g., tetracycline, neomycin, ampicillin, and kanamycin) may be contained, if desired. The pH of the medium is, for example, approximately 5 to 8. When the host cell is *E. coli,* for example, LB medium or M9 medium (Molecular Cloning, Cold Spring Harbor Laboratory, Vol. 3, A2.2) can be used as the medium. The culture is usually performed at approximately 14 to 43°C for approximately 3 to 24 hours, if necessary, with aeration or stirring. When the host cell is a yeast, for example, Burkholder minimum medium (Proc. Natl. Acad. Sci. USA., 1980, Vol. 77, p. 4505) can be used as the medium. The culture is usually performed at approximately 20 to 35°C for approximately 14 to 144 hours, if necessary, with aeration or stirring. The polypeptide of interest can be expressed by the culture as mentioned above.

The method may further comprise the step of adding or expressing sialyltransferase. Examples of such an enzyme include β-galactoside α-2,6-sialyltransferase 1 (ST6GAL1), sialyltransferase, and β-galactoside α-2,3-sialyltransferase. This further increases the amount of sialic acid added to the Fc region. The sialyltransferase may be simply added, or an expression vector having an insert of DNA encoding this enzyme may be prepared such that the enzyme is co-expressed with the Fc region variant of the present invention or the polypeptide comprising the Fc region variant of the present invention, such as an antibody. In the case of adding the sialyltransferase, the timing or the method of addition is not particularly limited. The sialyltransferase may be added, for example, in the course of polypeptide production by cell culture or may be added and reacted after polypeptide purification. In the case of expressing the sialyltransferase, examples thereof include, but are not limited to, a method of allowing cells to co-express a plasmid encoding the sialyltransferase and a plasmid encoding the polypeptide to produce a sialylated polypeptide. In the case of producing the polypeptide using cells containing endogenous glycosyltransferase, such as CHO cells, sialic acid can be added in the course of polypeptide production without the addition of the sialyltransferase as mentioned above.

The method according to an embodiment may further comprise the step of recovering, isolating, or purifying the polypeptide from the host cell, in addition to the step of culturing the transformed host cell so that the polypeptide comprising the sialylated Fc region variant is expressed. Examples of the isolation or purification method include methods that exploit solubility, such as salting-out and solvent precipitation, methods that exploit difference in molecular weight, such as dialysis, ultrafiltration, and gel filtration, methods that exploit charge, such as ion exchange chromatography and hydroxyapatite chromatography, methods that exploit specific affinity, such as affinity chromatography, methods that exploit difference in hydrophobicity, such as reverse-phase high-performance liquid chromatography, and methods that exploit difference in isoelectric point, such as isoelectric focusing electrophoresis. In one embodiment, the polypeptide secreted into the culture supernatant can be purified by each chromatography, for example, column chromatography using a protein A column or a protein G column.

According to one embodiment, the present invention provides a polypeptide comprising the sialylated Fc region variant, the polypeptide being produced by the production method mentioned above.


7. Pharmaceutical composition


According to one embodiment, the present invention provides a pharmaceutical composition comprising the sialylated Fc region variant of the present invention or the polypeptide comprising the sialylated Fc region variant mentioned above, and a pharmaceutically acceptable carrier. The pharmaceutical composition can be prepared by a method usually used using an excipient usually used in the art, i.e., a pharmaceutical excipient, a pharmaceutical carrier, or the like. Examples of the dosage form of the pharmaceutical composition include parenteral formulations such as injections and formulations for intravenous drips. Administration such as intravenous administration, subcutaneous administration, or intraperitoneal administration can be performed. For the production of preparations, excipients, carriers, additives, and the like appropriate for these dosage forms can be used in a pharmaceutically acceptable range.

The pharmaceutical composition according to an embodiment may further comprise an additional antibody drug and/or one or more additional therapeutic agents. The pharmaceutical composition according to an embodiment may be used in combination (concurrently or sequentially) with an additional antibody drug and/or one or more additional therapeutic agents.

The amount of the polypeptide according to an embodiment in the pharmaceutical composition can be appropriately set depending on the severity of symptoms or the age of a patient, the dosage form of the preparation used, etc.

The polypeptide according to an embodiment and the pharmaceutical composition containing the same can be used in the treatment of autoimmune diseases and inflammatory diseases. According to another embodiment, the present invention provides a method for treating an autoimmune disease, comprising administering a therapeutically effective amount of the polypeptide according to an embodiment to a subject. According to a further embodiment, the present invention includes the polypeptide of the present invention for use in the treatment of an autoimmune disease. The present invention also provides use of the polypeptide according to an embodiment in the production of a pharmaceutical composition for the treatment of an autoimmune disease. The autoimmune disease to be treated is not particularly limited.


Examples


In Examples given below, the "sialylation rate" means the ratio of a sialic acid-containing sugar chain to all N-linked sugar chains present in the system. For antibodies produced in Examples given below, the N-linked sugar chain was found to be only a sugar chain linked to asparagine at position 297 by structural analysis. Therefore, the sialylation rate means the ratio of a sialic acid-containing sugar chain to all N-linked sugar chains (i.e., the sugar chain linked to asparagine at position 297) present in the system.


<Example 1: Preparation of E294Del mutant of anti-lysozyme antibody>


DNAs encoding the polypeptides of an anti-lysozyme antibody heavy chain and light chain were synthesized in accordance with a routine method and each inserted into pcDNA3.4-TOPO vector (Thermo Fisher Scientific Inc.) to prepare a heavy chain expression vector and a light chain expression vector. A gene encoding the sequence of an anti-lysozyme antibody heavy chain variable region was linked to a gene encoding human Igγ1 having an E294Del (Del = deletion) amino acid mutation, and the resulting DNA encoding the heavy chain polypeptide was inserted into pcDNA3.4-TOPO vector to prepare an expression vector for the heavy chain having the E294Del amino acid mutation. ExpiCHO-S cells (Thermo Fisher Scientific Inc., A29127) were transfected with the heavy chain expression vector and the light chain expression vector, or the expression vector for the heavy chain having the E294Del amino acid mutation and the light chain expression vector, and cultured. A culture supernatant was purified using MabSelect SuRe (GE Healthcare Japan Corp., 17-5438-02) in accordance with a routine method to obtain a purified antibody. The antibody having the human Igγ1 constant region (Fc non-variant) was designated as LYS_1c3-m1_h10. The antibody having the E294Del amino acid mutation in the human Igγ1 constant region was designated as LYS_E294De1_h1x. Table 1 shows the amino acid sequence and the nucleic acid sequence of each antibody.

[Table 1]

| Antibody name | Heavy chain amino acid | Heavy chain nucleic acid | Light chain amino acid | Light chain nucleic acid |
|---|---|---|---|---|
| LYS_1c3-m1_h10 | SEQ ID NO: 1 | SEQ ID NO: 2 | SEQ ID NO: 3 | SEQ ID NO: 4 |
| LYS_E294Del_h1x | SEQ ID NO: 5 | SEQ ID NO: 6 | SEQ ID NO: 3 | SEQ ID NO: 4 |

Antibodies having the E294Del mutation have been reported to exhibit attenuated binding activity against human FcγRs (WO2012/175751, Table 4). According to the previous report (e.g., CELL IMMUNOL., 2000, Vol. 200 (1), p. 16-26), the reduced binding activity against FcγRs suppresses the activation of T cells and presumably can thereby reduce adverse reactions of the treatment of certain conditions.

Accordingly, the antibodies thus obtained were evaluated for their binding activity against human FcγRs using Biacore (GE Healthcare Japan Corp., Biacore T200) in accordance with the manual. The binding activity against human FcγRI still remained in LYS_E294Del_h1x, though the binding activity was attenuated as compared with LYS_1c3-m1_h10.

<Example 2: Preparation of L234A/L235A/E294Del mutant of anti-lysozyme antibody>

An antibody having the E294Del mutation mentioned above and an L234A mutation and an L235A mutation in combination was prepared by a method described below. A gene encoding the sequence of an anti-lysozyme antibody heavy chain variable region was linked to a gene encoding human Igγl having L234A/L235A/E294Del amino acid mutations, and the resulting DNA encoding the heavy chain polypeptide was inserted into pcDNA3.4-TOPO vector to prepare an expression vector for the heavy chain having the L234A/L235A/E294Del amino acid mutations. ExpiCHO-S cells were transfected with the expression vector for the heavy chain having the L234A/L235A/E294Del amino acid mutations and the light chain expression vector prepared in Example 1, and cultured. An antibody was purified by the same method as in Example 1. The obtained antibody, i.e., the antibody having the L234A/L235A/E294Del amino acid mutations in the human Igγ1 constant region, was designated as LYS_E294Del_LALA_h1x. Table 2 shows the amino acid sequence and the nucleic acid sequence of the antibody.

[Table 2]

| Antibody name | Heavy chain amino acid | Heavy chain nucleic acid | Light chain amino acid | Light chain nucleic acid |
|---|---|---|---|---|
| LYS_E294Del_LALA_hlx | SEQ ID NO: 7 | SEQ ID NO: 8 | SEQ ID NO: 3 | SEQ ID NO: 4 |

LYS_E294Del_LALA_h1x was evaluated for its binding activity against human FcγRI by the same method as in Example 1 using Biacore. As a result, the binding activity of LYS_E294Del_LALA_h1x against human FcγRI disappeared. Such an antibody from which the binding activity against human FcγRI disappeared is useful in exerting therapeutic effects while reducing adverse reactions in the treatment of certain conditions.

<Example 3: Preparation of sialylated anti-lysozyme antibody mutant and measurement of sialylation rate>

A mutant of the anti-lysozyme antibody having a sialic acid added thereto was prepared by a method described below using CHO cells, which are often used in industrial antibody production. The CHO cells express no $\alpha2,6$ sialyltransferase (MAbs, 2015, Vol. 7 (3), p. 571-83). Therefore, for the purpose of imparting $\alpha2,6$-linked sialic acid to the antibody, DNA encoding the polypeptide of $\beta$-galactoside $\alpha$-2,6-sialyltransferase 1 (ST6GAL1) (Uniprot: P15907-1) was inserted into pcDNA3.4-TOPO vector to prepare an expression vector.

ExpiCHO-S cells were transfected with the ST6GAL1 expression vector, together with the expression vector for the heavy chain having the E294Del amino acid mutation prepared in Example 1 and the light chain expression vector prepared in Example 1, or the expression vector for the heavy chain having the L234A/L235A/E294Del amino acid mutations prepared in Example 2 and the light chain expression vector prepared in Example 1 so that ST6GAL1 and each antibody were co-expressed. The amount of each expression vector added was adjusted such that the ratio of the expression vector encoding ST6GAL1 to the total of the heavy chain expression vector and the light chain expression vector was 2%. Each antibody was purified by the same method as in Example 1. The antibody having the human Igγ1 constant region having the E294Del amino acid mutation was designated as LYS_E294Del_h1x-ST6_2%. The antibody having the human Igγ1 constant region having the L234A/L235A/E294Del amino acid mutations was designated as LYS_E294Del_LALA_h1x-ST6_2%.

To each antibody, dithiothreitol (Thermo Fisher Scientific Inc., A39255) was added, and incubated at 37°C for 1 hour to reduce an inter-chain disulfide bond. The sample thus reduced was injected into an HPLC system (Waters Corp.), separated through a reverse-phase column (Waters Corp., ACQUITY UPLC Protein BEH C4), and then introduced into a mass spectrometer (Waters Corp.). Sugar chain modifications were attributed to their respective types by comparing a theoretical molecular weight of the molecular weights of various sugar chains added to the molecular weight of the reduced antibody with the actually measured mass, and the proportion (%) of each sugar chain was calculated from an ion intensity ratio. The total proportion of sialic acid-containing sugar chains was defined as the "sialylation rate (%)". The sialylation rate was calculated according to the following expression.

Sialylation rate (%) = (Total MS intensity of an H chain modified with the sialic acid-linked sugar chain) / (Total MS intensity of all the H chains) $\times$ 100.

The results are shown in Table 3.

[Table 3]

| Antibody name | Sialylation rate (%) |
| --- | --- |
| LYS_E294Del_h1x-ST6_2% | 22.9 |
| LYS_E294Del_LALA_h1x-ST6_2% | 28.0 |

Unexpectedly, LYS_E294Del_LALA_h1x-ST6_2% had a higher sialylation rate than that of LYS_E294Del_h1x-ST6_2%. In short, the combination of the E294Del mutation and the L234A/L235A mutations enhanced the sialylation rate of the antibody as compared with the E294Del mutation.

<Example 4: Three-dimensional structure analysis of antibody>

In order to discuss the cause of the enhanced sialylation rate observed in Example 3, model structures were constructed for the antibody having the E294Del mutation, the antibody having the L234A/L235A/E294Del mutations, an antibody having L234A/L235E/E294Del mutations, and an antibody having L234F/L235Q/E294Del mutations, and analyzed for their three-dimensional structures. For the model structures, the full-length structure of IgG1 (PDB code: 1HZH) was selected in which the S-S bond of an IgG hinge region is observed, and a hydrophobic anchorage of L234/L235 is formed. IgG1 sugar chains in the full-length structure of IgG1 were replaced with sialic acid-attached sugar chains isolated from a sialylated Fc structure (PDB code: 4BYH). Then, each of the E294Del mutation, the L234A/L235A/E294Del mutations, the L234A/L235E/E294Del mutations, and the L234F/L235Q/E294Del mutations was introduced thereinto.

(1) IgG1 model structure

L234/L235 of IgG1 forms strong Van der Waals interaction between the chains of a dimer. An anchorage is formed through this interaction, and Fcγ receptor binds thereto (J. Biol. Chem., 2017, Vol. 292 (9), p. 3900-3908; and Nature, 2000, Vol. 406, p. 267-273). In the full-length structure of IgG1, L234/L235 and the sugar chain linked to the amino group of a side chain at N297 position come into contact between the CH2 domains of a dimer (Figure 1). Hence, the interaction of L234/L235 and the sugar chain is considered to contribute to the structural formation and stability of the CH2 dimer.

(2) Sialylated IgG1 model structure harboring E294Del mutation

As a result of analyzing the IgG1 model structure of the sialylated E294Del mutant, the E293 or E294Del mutant having a mutation that improves a sialylation rate had the deletion of only one residue and however, was found to shorten a loop including N297 positioned on the C-terminal side by 3 residues therefrom (a string constituted by amino acids; hereinafter, referred to as an "N297 loop"). The shortened N297 loop was confirmed to result in displacement at the spatial position of N297 so that the sugar chain linked to N297 was distorted (Figure 2). It is considered that this distortion facilitates the access of glycosyltransferase, or secondary conformational change in the whole CH2 enhances the sialylation rate.

Next, as a result of analyzing the dimer interface of L234/L235, the CH2 dimer interface sugar chain was distorted, whereas the strong Van der Waals interaction between L234 and L235 was still confirmed (Figure 3).

(3) Sialylated IgG1 model structure harboring L234A/L235A/E294Del mutations

As a result of preparing and analyzing the E294-deletion and L234A/L235A mutant model structure, the L234A/L235A Van der Waals interaction was confirmed to be weaker than the L234/L235 interaction, so that space was created between these sites (Figure 4). This change is considered to contribute to the improved sialylation rate.

(4) Sialylated IgG1 model structure harboring L234A/L235E/E294Del mutations

As a result of preparing and analyzing the E294-deletion and L234A/L235E mutant model structure, alanine (A) and glutamic acid (E) faced each other between L234A and L235E, and the L234A/L235E Van der Waals interaction was confirmed to be thereby weakened so that space was created between these sites (Figure 5). As in the antibody having the L234A/L235A/E294Del mutations, this change is considered to contribute to the improved sialylation rate.

(5) Sialylated IgG1 model structure harboring L234F/L235Q/E294Del mutations

As a result of preparing and analyzing the E294-deletion and L234F/L235Q mutant model structure, phenylalanine (F) and glutamine (Q) faced each other between L234F and L235Q, and the L234F/L235Q Van der Waals interaction was confirmed to be thereby weakened so that space was created between these sites (Figure 6). As in the antibody having the L234A/L235A/E294Del mutations, this change is considered to contribute to the improved sialylation rate.

(6) Discussion on sialylation rate improving mutation

The E294-deletion and L234A/L235A mutations enhanced the sialylation rate as compared with the E294-deletion mutation alone, presumably because the L234/L235 Van der Waals interaction of a dimer was weakened. Hence, the E294 deletion and the change of L234/L235 to less hydrophobic amino acids than leucine (L) or the change of the L234/L235 residues facing each other to a hydrophobic/hydrophilic combination are considered to weaken the Van der Waals interaction and to enhance the sialylation rate.

In addition to the Van der Waals interaction, the change of L234/L235 to smaller amino acids than L serves as a mutation that disrupts the anchorage formation between L234 and L235 and is also considered to enhance the sialylation rate.

<Example 5: Preparation of anti-fibronectin extra domain-A isoform (Fn-EDA) antibody>

DNA encoding a heavy chain polypeptide having the E294Del or L234A/L235A/E294Del amino acid mutations added to the sequence of an anti-fibronectin extra domain-A isoform (Fn-EDA) antibody heavy chain was inserted into pcDNA3.4-TOPO vector to prepare an expression vector for the heavy chain having the E294Del amino acid mutation, and an expression vector for the heavy chain having the L234A/L235A/E294Del amino acid mutations. DNA encoding the light chain polypeptide of the anti-Fn-EDA antibody was synthesized in accordance with a routine method and inserted into pcDNA3.4-TOPO vector to prepare a light chain expression vector.

ExpiCHO-S cells were transfected with the ST6GAL1 expression vector prepared in Example 3, together with the expression vector for the heavy chain having the E294Del amino acid mutation and the light chain expression vector, or the expression vector for the heavy chain having the L234A/L235A/E294Del amino acid mutations and the light chain expression vector so that ST6GAL1 and each antibody were co-expressed. The amount of each expression vector added was adjusted such that the ratio of the expression vector encoding ST6GAL1 to the total of the heavy chain expression vector and the light chain expression vector was 0.5%, 1%, 2%, and 5%. Each antibody was purified by the same method as in Example 1.

The antibody having the human Igγ1 constant region having the E294Del amino acid mutation was designated as GLY_F8_E294Del_h1x, which was designated as GLY_F8_E294Del_h1x (0.5%), GLY_F8_E294Del_h1x (1%), GLY_F8_E294Del_h1x (2%), and GLY_F8_E294Del_h1x (5%) depending on the amount of ST6GAL1 co-expressed. The antibody having the human Igγ1 constant region having the L234A/L235A/E294Del amino acid mutations was designated as GLY_F8_E294Del_LALA_h1x, which was designated as GLY_F8_E294Del_LALA_h1x (0.5%), GLY_F8_E294Del_LALA_h1x (1%), GLY_F8_E294Del_LALA_h1x (2%), and GLY_F8_E294Del_LALA_h1x (5%) depending on the amount of ST6GAL1 co-expressed. Table 4 shows the amino acid sequence and the nucleic acid sequence of each antibody.

[Table 4]

| Antibody name | Heavy chain amino acid | Heavy chain nucleic acid | Light chain amino acid | Light chain nucleic acid |
|---|---|---|---|---|
| GLY_F8_E294Del_hlx | SEQ ID NO: 9 | SEQ ID NO: 10 | SEQ ID NO: 11 | SEQ ID NO: 12 |
| GLY_F8_E294Del_LALA_h1x | SEQ ID NO: 13 | SEQ ID NO: 14 | SEQ ID NO: 11 | SEQ ID NO: 12 |

Each antibody was analyzed for its sialylation rate by the method described in Example 3. The results are shown in Table 5.

[Table 5]

| Antibody name | Sialylation rate (%) |
|---|---|
| GLY_F8_E294Del_h1x(0.5%) | 5.3 |
| GLY_F8_E294Del_LALA_h1x(0.5%) | 8.3 |
| GLY_F8_E294Del_h1x(1%) | 6.8 |
| GLY_F8_E294Del_LALA_h1x(1%) | 10.5 |
| GLY_F8_E294Del_h1x(2%) | 7.0 |
| GLY_F8_E294Del_LALA_h1x(2%) | 10.8 |
| GLY_F8_E294Del _h1x(5%) | 5.9 |
| GLY_F8_E294Del_LALA_h1x(5%) | 10.4 |

Under all the ST6GAL1 co-expression conditions, the anti-Fn-EDA antibody having the L234A/L235A/E294Del mutations exhibited a higher sialylation rate than that of the anti-Fn-EDA antibody having the E294Del mutation.

<Example 6: Preparation of anti-Fn-EDA variant antibody>

The heavy chain and light chain framework (FR) regions of each anti-Fn-EDA antibody obtained in Example 5 were replaced with the FR sequences of another human antibody known in the art. For the purpose of reducing latent glycation risk, a K94R mutation was further introduced into the sequence of the heavy chain variable region to prepare an anti-Fn-EDA variant antibody. A specific preparation method is as follows.

DNAs encoding the polypeptides of an anti-Fn-EDA variant antibody heavy chain and light chain were synthesized in accordance with a routine method and each inserted into pcDNA3.4-TOPO vector to prepare a heavy chain expression vector and a light chain expression vector. A gene encoding the sequence of an anti-Fn-EDA variant antibody heavy chain variable region was linked to a gene encoding human Igyl having L234A/L235A, E294Del, or L234A/L235A/E294Del amino acid mutations, and the resulting DNA encoding the heavy chain polypeptide was inserted into pcDNA3.4-TOPO vector to prepare an expression vector for the heavy chain having the L234A/L235A amino acid mutations, an expression vector for the heavy chain having the E294Del amino acid mutation, and an expression vector for the heavy chain having the L234A/L235A/E294Del amino acid mutations.

Next, ExpiCHO-S(R) cells were transfected with the expression vector encoding ST6GAL1 prepared in Example 3, together with the heavy chain expression vector and the light chain expression vector, the expression vector for the heavy chain having the L234A/L235A amino acid mutations and the light chain expression vector, the expression vector for the heavy chain having the E294Del amino acid mutation and the light chain expression vector, or the expression vector for the heavy chain having the L234A/L235A/E294Del amino acid mutations and the light chain expression vector, obtained as described above so that ST6GAL1 and each antibody were co-expressed. The amount of each expression vector added was adjusted such that the ratio of the expression vector encoding ST6GAL1 to the total of the heavy chain expression vector and the light chain expression vector was 5%.

Then, each antibody was purified by the same method as in Example 1. The antibody having the human Igy1 constant region (Fc non-variant) was designated as SSA_m3_K94R_h10. The antibody having the L234A/L235A amino acid mutations in the human Igy1 constant region was designated as SSA_m3_K94R_h1g. The antibody having the E294Del amino acid mutation in the human Igγ1 constant region was designated as SSA_E294Del_m3_K94R_h1x. The antibody having the L234A/L235A/E294Del amino acid mutations in the human Igγ1 constant region was designated as SSA_E294Del_LALA_m3_K94R_h1x. Table 6 shows the amino acid sequence and the nucleic acid sequence of each antibody.

[Table 6]

| Antibody name | Heavy chain amino acid | Heavy chain nucleic acid | Light chain amino acid | Light chain nucleic acid |
|---|---|---|---|---|
| SSA_m3_K94R_h10 | SEQ ID NO: 15 | SEQ ID NO: 16 | SEQ ID NO: 17 | SEQ ID NO: 18 |
| SSA_m3_K94R_h1g | SEQ ID NO: 19 | SEQ ID NO: 20 | SEQ ID NO: 17 | SEQ ID NO: 18 |
| SSA_E294Del_m3_K94R_hlx | SEQ ID NO: 21 | SEQ ID NO: 22 | SEQ ID NO: 17 | SEQ ID NO: 18 |
| SSA_E294Del_LALA_m3_K94R_hlx | SEQ ID NO: 23 | SEQ ID NO: 24 | SEQ ID NO: 17 | SEQ ID NO: 18 |

Each antibody was analyzed for its sialylation rate by the method described in Example 3. The results are shown in Table 7.

[Table 7]

| Antibody name | Sialylation rate (%) |
|---|---|
| SSA_m3_K94R_h10 | 1.3 |
| SSA_m3_K94R_h1g | 0.0 |
| SSA_E294Del_m3_K94R_h1x | 16.5 |
| SSA_E294Del_LALA_m3_K94R_hlx | 21.9 |

Sialic acid was rarely imparted to the Fc non-variant and the antibody having the L234A/L235A mutations, whereas the sialylation rate was enhanced in the antibody having the E294Del mutation. The sialylation rate was further enhanced in the antibody having the E294Del mutation and the L234A/L235A mutations, as compared with the antibody having the E294Del mutation.

When the anti-Fn-EDA antibodies which were obtained in Example 5 and prepared under the same conditions as above (i.e., GLY_F8_E294Del_h1x (5%) and GLY_F8_E294Del_LALA_h1x (5%)) were compared with the anti-Fn-EDA variant antibodies obtained in this Example (i.e., SSA_E294Del_m3_K94R_h1x and SSA_E294Del_LALA_m3_K94R_h1x), the anti-Fn-EDA variant antibodies exhibited a higher sialylation rate among all the antibodies having the E294Del mutation and the antibodies having the E294Del mutation and the L234A/L235A mutations. These results indicate that the conversion of the FR sequences can enhance the sialylation rate of the Fc region.

The amino acid sequences and DNA sequences of the polypeptides according to an embodiment are shown in the following tables.

[Table 8-1]

| Table 8 | | | | |
|---|---|---|---|---|
| SEQ ID NO: | Sequence name | Organism from which sequence is derived | DNA/prot ein | Sequence information |
| 1 | anti-LYS_H chain | Human | Protein | QVQLVQSGAEVKKPGASVKVSCKASGYAFSSSWVNWVRQAPGQGLEWMGRIYPGDGD TDYNGKFKGRVTLTADKSTSTAYMELSSLRSEDTAVYYCARRRGYEAVFDYWGQGTLV TVSSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPAPELL GGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPS RDELTKNQVSLTCLVKGFYPSDIAVEWESNGQPENNYKTTPPVLDSDGSFFLYSKLTVDK SRWQQGNVFSCSVMHEALHNHYTQKSLSLSPGK |

[Table 8-2]

| Table 8 (continued) | | | | |
|---|---|---|---|---|
| 2 | anti-LYS_H chain | Human | DNA | CAGGTGCAGCTGGTTCAGTCTGGCGCCGAAGTGAAGAAACCTGGCGCCTCTGTGAAG GTGTCCTGCAAGGCTTCTGGCTACGCCTTCTCCTCCTCCTGGGTCAACTGGGTCCGAC AGGCTCCTGGACAAGGCCTGGAATGGATGGGCAGAATCTATCCTGGCGACGGCGACA CCGACTACAACGGCAAGTTTAAGGGCAGAGTGACCCTGACCGCCGACAAGTCTACCT CCACCGCCTACATGGAACTGTCCAGCCTGAGATCTGAGGACACCGCCGTGTACTACTG CGCTCGGAGAAGAGGCTACGAGGCCGTGTTTGATTATTGGGGCCAGGGCACCCTGGT CACCGTGTCCTCTGCTTCTACCAAGGGACCCAGCGTGTTCCCTCTGGCTCCTAGCTCTA AGTCCACCTCTGGCGGAACAGCTGCTCTGGGCTGTCTGGTCAAGGACTACTTCCCTGA GCCTGTGACCGTGTCTTGGAACTCTGGCGCTCTGACATCCGGCGTGCACACCTTTCCA GCTGTGCTGCAATCCTCCGGCCTGTACTCTCTGTCCTCCGTCGTGACCGTGCCTTCTAG CTCTCTGGGCACCCAGACCTACATCTGCAATGTGAACCACAAGCCTTCCAACACCAAG GTGGACAAGAAGGTGGAACCCAAGTCCTGCGACAAGACCCACACCTGTCCTCCATGT CCTGCTCCAGAACTGCTCGGCGGACCTTCCGTGTTTCTGTTCCCTCCAAAGCCTAAGG ACACCCTGATGATCTCTCGGACCCCTGAAGTGACCTGCGTGGTGGTGGATGTGTCTCA CGAGGACCCAGAAGTGAAGTTCAATTGGTACGTGGACGGCGTGGAAGTGCACAACGC CAAGACCAAGCCTAGAGAGGAACAGTACAACTCCACCTACAGAGTGGTGTCCGTGCT GACCGTGCTGCACCAGGATTGGCTGAACGGCAAAGAGTACAAGTGCAAGGTGTCCAA CAAGGCCCTGCCTGCTCCTATCGAAAAGACCATCTCCAAGGCCAAGGGCCAGCCTAG GGAACCCCAGGTTTACACCTTGCCTCCATCTCGGGACGAGCTGACCAAGAACCAGGT GTCCCTGACCTGTCTCGTGAAGGGCTTCTACCCCTCCGATATCGCCGTGGAATGGGAG TCTAATGGCCAGCCAGAGAACAACTACAAGACAACCCCTCCTGTGCTGGACTCCGAC GGCTCATTCTTCCTGTACTCCAAGCTGACAGTGGACAAGTCCAGATGGCAGCAGGGC AACGTGTTCTCCTGCTCCGTGATGCACGAGGCCCTGCACAATCACTACACACAGAAGT CCCTGTCTCTGTCCCCTGGCAAG |

[Table 8-3]

| Table 8 (continued) | | | | |
|---|---|---|---|---|
| 3 | anti-LYS_L chain | Human | Protein | DIQLTQSPSSLSASVGDRVTITCRASESVDNYDISFLNWFQQKPGKAPKLLIYAASNQGSG VPSRFSGSGSGTDFTLTISSLQPEDFATYYCQQSKEVPYTFGGGTKVEIKRTVAAPSVFIFPP SDEQLKSGTASVVCLLNNFYPREAKVQWKVDNALQSGNSQESVTEQDSKDSTYSLSSTLT LSKADYEKHKVYACEVTHQGLSSPVTKSFNRGEC |
| 4 | anti-LYS_L chain | Human | DNA | GACATCCAGCTGACCCAGAGCCCTTCCAGCCTGTCTGCTTCCGTGGGCGACAGAGTGA CCATCACCTGTCGGGCCTCCGAGTCCGTGGACAACTACGATATCTCCTTCCTGAACTG GTTCCAGCAGAAGCCCGGCAAGGCCCCCAAGCTGCTGATCTACGCCGCCTCCAATCA GGGCTCCGGCGTGCCCTCTAGATTCTCCGGCTCTGGCTCTGGCACCGACTTTACCCTG ACCATCTCCAGCCTGCAGCCCGAGGACTTCGCCACCTACTACTGCCAGCAGTCCAAAG AGGTGCCCTACACCTTCGGCGGAGGCACCAAGGTGGAAATCAAGCGGACCGTGGCCG CTCCCTCCGTGTTCATCTTCCCACCTTCCGACGAGCAGCTGAAGTCCGGCACCGCTTCT GTCGTGTGCCTGCTGAACAACTTCTACCCCCGCGAGGCCAAGGTGCAGTGGAAGGTG GACAACGCCCTGCAGTCCGGCAACTCCCAGGAATCCGTGACCGAGCAGGACTCCAAG GACAGCACCTACTCCCTGTCCTCCACCCTGACCCTGTCCAAGGCCGACTACGAGAAGC ACAAGGTGTACGCCTGCGAAGTGACCCACCAGGGCCTGTCTAGCCCCGTGACCAAGT CTTTCAACCGGGGCGAGTGC |
| 5 | anti-LYS_H chain_E294 Del | Human | Protein | QVQLVQSGAEVKKPGASVKVSCKASGYAFSSSWVNWVRQAPGQGLEWMGRIYPGDGD TDYNGKFKGRVTLTADKSTSTAYMELSSLRSEDTAVYYCARRRGYEAVFDYWGQGTLV TVSSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPAPELL GGPSVFLFPPKPKDTLMISRTPEVTCVVVDVSHEDPEVKFNWYVDGVEVHNAKTKPREQ YNSTYRVVSVLTVLHQDWLNGKEYKCKVSNKALPAPIEKTISKAKGQPREPQVYTLPPSR DELTKNQVSLTCLVKGFYPSDIAVEWESNGQPENNYKTTPPVLDSDGSFFLYSKLTVDKS RWQQGNVFSCSVMHEALHNHYTQKSLSLSPGK |

EP 4 772 534 A1

[Table 8-4]

Table 8 (continued)

| 6 | anti-LYS_H chain_E294 Del | Human | DNA | CAGGTGCAGCTGGTTCAGTCTGGCGCCGAAGTGAAGAAACCTGGCGCCTCTGTGAAG GTGTCCTGCAAGGCTTCTGGCTACGCCTTCTCCTCCTCCTGGGTCAACTGGGTCCGAC AGGCTCCTGGACAAGGCCTGGAATGGATGGGCAGAATCTATCCTGGCGACGGCGACA CCGACTACAACGGCAAGTTTAAGGGCAGAGTGACCCTGACCGCCGACAAGTCTACCT CCACCGCCTACATGGAACTGTCCAGCCTGAGATCTGAGGACACCGCCGTGTACTACTG CGCTCGGAGAAGAGGCTACGAGGCCGTGTTTGATTATTGGGGCCAGGGCACCCTGGT CACCGTGTCCTCTGCTTCTACCAAGGGACCCAGCGTGTTCCCTCTGGCTCCTAGCTCTA AGTCCACCTCTGGCGGAACAGCTGCTCTGGGCTGTCTGGTCAAGGACTACTTCCCTGA GCCTGTGACCGTGTCTTGGAACTCTGGCGCTCTGACATCCGGCGTGCACACCTTTCCA GCTGTGCTGCAATCCTCCGGCCTGTACTCTCTGTCCTCCGTCGTGACCGTGCCTTCTAG CTCTCTGGGCACCCAGACCTACATCTGCAATGTGAACCACAAGCCTTCCAACACCAAG GTGGACAAGAAGGTGGAACCCAAGTCCTGCGACAAGACCCACACCTGTCCTCCATGT CCTGCTCCAGAACTGCTCGGCGGACCTTCCGTGTTTCTGTTCCCTCCAAAGCCTAAGG ACACCCTGATGATCTCTCGGACCCCTGAAGTGACCTGCGTGGTGGTGGATGTGTCTCA CGAGGACCCAGAAGTGAAGTTCAATTGGTACGTGGACGGCGTGGAAGTGCACAACGC CAAGACCAAGCCTAGAGAGCAGTACAACTCCACCTACAGAGTGGTGTCCGTGCTGAC CGTGCTGCACCAGGATTGGCTGAACGGCAAAGAGTACAAGTGCAAGGTGTCCAACAA GGCCCTGCCTGCTCCTATCGAAAAGACCATCTCCAAGGCCAAGGGCCAGCCTAGGGA ACCCCAGGTTTACACCTTGCCTCCATCTCGGGACGAGCTGACCAAGAACCAGGTGTCC CTGACCTGTCTCGTGAAGGGCTTCTACCCCTCCGATATCGCCGTGGAATGGGAGTCTA ATGGCCAGCCAGAGAACAACTACAAGACAACCCCTCCTGTGCTGGACTCCGACGGCT CATTCTTCCTGTACTCCAAGCTGACAGTGGACAAGTCCAGATGGCAGCAGGGCAACG TGTTCTCCTGCTCCGTGATGCACGAGGCCCTGCACAATCACTACACACAGAAGTCCCT GTCTCTGTCCCCTGGCAAG |

[Table 8-5]

Table 8 (continued)

| 7 | anti-LYS_H chain_L234A/ L235A/E294D el | Human | Protein | QVQLVQSGAEVKKPGASVKVSCKASGYAFSSSWVNWVRQAPGQGLEWMGRIYPGDGDTDYNGKF KGRVTLTADKSTSTAYMELSSLRSEDTAVYYCARRRGYEAVFDYWGQGTLVTVSSASTKGPSVFPL APSSKSTSGGTAALGCLVKDYFPEPVTVSWNSGALTSGVHTFPAVLQSSGLYSLSSVVTVPSSSLGTQ TYICNVNHKPSNTKVDKKVEPKSCDKTHTCPPCPAPEAAGGPSVFLFPPKPKDTLMISRTPEVTCVVV DVSHEDPEVKFNWYVDGVEVHNAKTKPREQYNSTYRVVSVLTVLHQDWLNGKEYKCKVSNKALP APIEKTISKAKGQPREPQVYTLPPSRDELTKNQVSLTCLVKGFYPSDIAVEWESNGQPENNYKTTPPV LDSDGSFFLYSKLTVDKSRWQQGNVFSCSVMHEALHNHYTQKSLSLSPGK |
| 8 | anti-LYS_H chain_L234A/ L235A/E294D el | Human | DNA | CAGGTGCAGCTGGTTCAGTCTGGCGCCGAAGTGAAGAAACCTGGCGCCTCTGTGAAGGTGTCCT GCAAGGCTTCTGGCTACGCCTTCTCCTCCTCCTGGGTCAACTGGGTCCGACAGGCTCCTGGACAA GGCCTGGAATGGATGGGCAGAATCTATCCTGGCGACGGCGACACCGACTACAACGGCAAGTTTA AGGGCAGAGTGACCCTGACCGCCGACAAGTCTACCTCCACCGCCTACATGGAACTGTCCAGCCT GAGATCTGAGGACACCGCCGTGTACTACTGCGCTCGGAGAAGAGGCTACGAGGCCGTGTTTGAT TATTGGGGCCAGGGCACCCTGGTCACCGTGTCCTCTGCTTCTACCAAGGGACCCAGCGTGTTCCC TCTGGCTCCTAGCTCTAAGTCCACCTCTGGCGGAACAGCTGCTCTGGGCTGTCTGGTCAAGGACT ACTTCCCTGAGCCTGTGACCGTGTCTTGGAACTCTGGCGCTCTGACATCCGGCGTGCACACCTTT CCAGCTGTGCTGCAATCCTCCGGCCTGTACTCTCTGTCCTCCGTCGTGACCGTGCCTTCTAGCTCT CTGGGCACCCAGACCTACATCTGCAATGTGAACCACAAGCCTTCCAACACCAAGGTGGACAAGA AGGTGGAACCCAAGTCCTGCGACAAGACCCACACCTGTCCTCCATGTCCTGCTCCAGAAGCTGC TGGCGGCCCTTCCGTGTTTCTGTTCCCTCCAAAGCCTAAGGACACCCTGATGATCTCTCGGACCC CTGAAGTGACCTGCGTGGTGGTGGATGTGTCTCACGAGGACCCAGAAGTGAAGTTCAATTGGTA CGTGGACGGCGTGGAAGTGCACAACGCCAAGACCAAGCCTAGAGAGCAGTACAACTCCACCTA CAGAGTGGTGTCCGTGCTGACCGTGCTGCACCAGGATTGGCTGAACGGCAAAGAGTACAAGTGC AAGGTGTCCAACAAGGCCCTGCCTGCTCCTATCGAAAAGACCATCTCCAAGGCCAAGGGCCAGC CTAGGGAACCCCAGGTTTACACCTTGCCTCCATCTCGGGACGAGCTGACCAAGAACCAGGTGTC CCTGACCTGTCTCGTGAAGGGCTTCTACCCCTCCGATATCGCCGTGGAATGGGAGTCTAATGGCC AGCCAGAGAACAACTACAAGACAACCCCTCCTGTGCTGGACTCCGACGGCTCATTCTTCCTGTAC TCCAAGCTGACAGTGGACAAGTCCAGATGGCAGCAGGGCAACGTGTTCTCCTGCTCCGTGATGC ACGAGGCCCTGCACAATCACTACACACAGAAGTCCCTGTCTCTGTCCCCTGGCAAG |

EP 4 772 534 A1

[Table 8-6]

| Table 8 (continued) | | | | |
|---|---|---|---|---|
| 9 | anti-Fn EDA_H chain_ E294De 1 | Human | Protein | EVQLVESGGGLVQPGGSLRLSCAASGFTFSLFTMSWVRQAPGKGLEWVSAISGSGGSTYYADSVK GRFTISRDNSKNTLYLQMNSLRAEDTAVYYCAKSTHLYLFDYWGQGTLVTVSSASTKGPSVFPLAP SSKSTSGGTAALGCLVKDYFPEPVTVSWNSGALTSGVHTFPAVLQSSGLYSLSSVVTVPSSSLGTQT YICNVNHKPSNTKVDKKVEPKSCDKTHTCPPCPAPELLGGPSVFLFPPKPKDTLMISRTPEVTCVVV DVSHEDPEVKFNWYVDGVEVHNAKTKPREQYNSTYRVVSVLTVLHQDWLNGKEYKCKVSNKAL PAPIEKTISKAKGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPGK |
| 10 | anti-Fn EDA_H chain_E294De 1 | Human | DNA | GAAGTTCAACTCGTGGAATCCGGCGGCGGCCTGGTTCAGCCAGGTGGCAGCCTCCGACTCTCA TGTGCAGCCAGTGGATTCACATTCAGTCTTTTCACCATGTCTTGGGTTAGGCAGGCTCCCGGAA AGGGCCTGGAATGGGTTTCAGCAATCAGCGGATCAGGTGGCAGTACCTACTACGCTGATAGCG TCAAGGGCCGTTTCACAATCAGTCGGGACAATAGCAAGAATACTCTTTATCTTCAAATGAATA GCCTGCGCGCAGAAGATACAGCCGTCTACTATTGCGCAAAGTCAACTCATCTCTACCTCTTTGA TTATTGGGGACAGGGCACCCTTGTGACAGTAAGCTCAGCCTCCACAAAAGGTCCTTCTGTGTTT CCTCTCGCTCCATCTTCCAAATCAACCTCAGGTGGCACAGCCGCCCTTGGGTGCCTCGTTAAAG ATTACTTCCCTGAACCTGTTACCGTCAGCTGGAACAGCGGAGCCCTGACCTCCGGAGTCCACAC TTTCCCAGCTGTTCTGCAGAGCAGTGGCCTCTACTCACTCAGCAGTGTTGTGACTGTCCCTTCA AGTTCTTTGGGTACACAAACTTATATCTGCAACGTTAACCACAAACCATCCAACACCAAAGTCG ATAAGAAGGTCGAGCCTAAGTCCTGCGACAAAACTCATACTTGCCCACCCTGCCCCGCACCAG AGCTCTTGGGAGGTCCAAGCGTCTTTCTCTTCCCTCCTAAACCCAAGGATACACTTATGATATC AAGAACCCCTGAGGTCACATGTGTCGTCGTCGACGTATCACATGAGGACCCTGAGGTGAAATT CAATTGGTACGTAGATGGCGTCGAAGTCCATAATGCTAAGACCAAACCTCGAGAACAGTATAA CAGTACTTACCGAGTAGTTTCAGTCTTGACTGTGTTGCACCAGGACTGGCTCAACGGGAAGGA ATACAAATGCAAAGTTTCTAACAAGGCTTTGCCTGCTCCAATTGAGAAAACTATTAGCAAGGC CAAGGGTCAGCCCCGTGAGCCACAAGTATATACTCTTCCTCCTTCAAGGGACGAATTGACCAA GAACCAGGTTTCCTTGACTTGCTTGGTTAAAGGATTCTACCCCAGTGACATCGCAGTGGAATGG GAAAGTAACGGGCAGCCTGAGAATAATTACAAGACTACTCCTCCTGTGCTTGATTCAGATGGA AGTTTCTTCCTTTACAGTAAGTTGACTGTAGACAAAAGTCGATGGCAACAAGGGAATGTGTTTT CTTGTTCTGTGATGCATGAGGCATTGCATAACCATTATACACAGAAATCCCTGTCATTGTCCCC AGGTAAA |

EP 4 772 534 A1

[Table 8-7]

| Table 8 (continued) | | | | |
|---|---|---|---|---|
| 11 | anti-Fn EDA_L chain | Human | Protein | EIVLTQSPGTLSLSPGEKATLSCRASQSVSMPFLAWYQQKPGQAPRLLIYGASSRATGIPD RFSGSGSGTDFTLTISRLEPEDFAVYYCQQMRGRPPTFGQGTKVEIKRTVAAPSVFIFPPSD EQLKSGTASVVCLLNNFYPREAKVQWKVDNALQSGNSQESVTEQDSKDSTYSLSSTLTLS KADYEKHKVYACEVTHQGLSSPVTKSFNRGEC |
| 12 | anti-Fn EDA_L chain | Human | DNA | GAAATAGTATTGACCCAGTCACCTGGTACACTGTCACTGTCTCCAGGAGAAAAGGCA ACCTTGTCATGCCGGGCAAGCCAGTCTGTGTCCATGCCTTTCTTGGCATGGTACCAGC AGAAGCCAGGTCAAGCTCCCAGGCTGCTTATCTATGGAGCTAGCTCTAGGGCTACCG GTATTCCTGACCGGTTCAGCGGCTCAGGCTCTGGGACCGATTTCACACTCACCATTAG CAGACTGGAACCTGAAGACTTTGCTGTCTACTACTGCCAGCAGATGCGAGGAAGACC TCCCACCTTCGGTCAGGGTACTAAAGTGGAGATAAAGAGAACCGTCGCTGCTCCCTCT GTATTCATATTTCCTCCAAGTGATGAACAGCTGAAGTCCGGCACTGCCTCTGTGGTTT GCCTGCTCAACAACTTTTACCCACGCGAGGCCAAGGTGCAGTGGAAGGTCGACAATG CTCTGCAGAGTGGAAATTCACAGGAAAGCGTGACAGAACAAGATTCCAAGGACTCCA CATATAGCCTTAGTAGTACTCTGACTCTGTCCAAAGCCGATTATGAGAAACATAAAGT ATATGCCTGTGAGGTTACTCACCAGGGGCTGTCCAGTCCCGTGACAAAATCATTTAAC CGCGGCGAGTGC |
| 13 | anti-Fn EDA_H chain_L234 A/L235A/E2 94Del | Human | Protein | EVQLVESGGGLVQPGGSLRLSCAASGFTFSLFTMSWVRQAPGKGLEWVSAISGSGGSTYY ADSVKGRFTISRDNSKNTLYLQMNSLRAEDTAVYYCAKSTHLYLFDYWGQGTLVTVSSA STKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPPCPAPEAAGGPSV FLFPPKPKDTLMISRTPEVTCVVVDVSHEDPEVKFNWYVDGVEVHNAKTKPREQYNSTY RVVSVLTVLHQDWLNGKEYKCKVSNKALPAPIEKTISKAKGQPREPQVYTLPPSRDELTK NQVSLTCLVKGFYPSDIAVEWESNGQPENNYKTTPPVLDSDGSFFLYSKLTVDKSRWQQ GNVFSCSVMHEALHNHYTQKSLSLSPGK |

EP 4 772 534 A1

[Table 8-8]

| 14 | anti-Fn EDA_H chain_L234 A/L235A/E2 94Del | Human | DNA | GAAGTTCAACTCGTGGAATCCGGCGGCGGCCTGGTTCAGCCAGGTGGCAGCCTCCGA CTCTCATGTGCAGCCAGTGGATTCACATTCAGTCTTTTCACCATGTCTTGGGTTAGGCA GGCTCCCGGAAAGGGCCTGGAATGGGTTTCAGCAATCAGCGGATCAGGTGGCAGTAC CTACTACGCTGATAGCGTCAAGGGCCGTTTCACAATCAGTCGGGACAATAGCAAGAA TACTCTTTATCTTCAAATGAATAGCCTGCGCGCAGAAGATACAGCCGTCTACTATTGC GCAAAGTCAACTCATCTCTACCTCTTTGATTATTGGGGACAGGGCACCCTTGTGACAG TAAGCTCAGCTTCTACCAAGGGACCCAGCGTGTTCCCTCTGGCTCCTAGCTCTAAGTC CACCTCTGGCGGAACAGCTGCTCTGGGCTGTCTGGTCAAGGACTACTTCCCTGAGCCT GTGACCGTGTCTTGGAACTCTGGCGCTCTGACATCCGGCGTGCACACCTTTCCAGCTG TGCTGCAATCCTCCGGCCTGTACTCTCTGTCCTCCGTCGTGACCGTGCCTTCTAGCTCT CTGGGCACCCAGACCTACATCTGCAATGTGAACCACAAGCCTTCCAACACCAAGGTG GACAAGAAGGTGGAACCCAAGTCCTGCGACAAGACCCACACCTGTCCTCCATGTCCT GCTCCAGAAGCTGCTGGCGGCCCTTCCGTGTTTCTGTTCCCTCCAAAGCCTAAGGACA CCCTGATGATCTCTCGGACCCCTGAAGTGACCTGCGTGGTGGTGGATGTGTCTCACGA GGACCCAGAAGTGAAGTTCAATTGGTACGTGGACGGCGTGGAAGTGCACAACGCCAA GACCAAGCCTAGAGAGCAGTACAACTCCACCTACAGAGTGGTGTCCGTGCTGACCGT GCTGCACCAGGATTGGCTGAACGGCAAAGAGTACAAGTGCAAGGTGTCCAACAAGGC CCTGCCTGCTCCTATCGAAAAGACCATCTCCAAGGCCAAGGGCCAGCCTAGGGAACC CCAGGTTTACACCTTGCCTCCATCTCGGGACGAGCTGACCAAGAACCAGGTGTCCCTG ACCTGTCTCGTGAAGGGCTTCTACCCCTCCGATATCGCCGTGGAATGGGAGTCTAATG GCCAGCCAGAGAACAACTACAAGACAACCCCTCCTGTGCTGGACTCCGACGGCTCAT TCTTCCTGTACTCCAAGCTGACAGTGGACAAGTCCAGATGGCAGCAGGGCAACGTGTT CTCCTGCTCCGTGATGCACGAGGCCCTGCACAATCACTACACACAGAAGTCCCTGTCT CTGTCCCCTGGCAAG |

EP 4 772 534 A1

26

[Table 8-9]

| Table 8 (continued) | | | | |
|---|---|---|---|---|
| 15 | anti-Fn EDA variant_H chain | Human | Protein | QVELVESGGGLVQPGGSLRLSCAASGFTFSLFTMSWVRQAPGKGLEWVSAISGSGGSTYYADSVK GRFTISRDNSKNTLYLQMNSLRAEDTAVYYCARSTHLYLFDYWGQGTLVTVSSASTKGPSVFPLAP SSKSTSGGTAALGCLVKDYFPEPVTVSWNSGALTSGVHTFPAVLQSSGLYSLSSVVTVPSSSLGTQT YICNVNHKPSNTKVDKKVEPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLNGKEYKCKVSNKA LPAPIEKTISKAKGQPREPQVYTLPPSRDELTKNQVSLTCLVKGFYPSDIAVEWESNGQPENNYKTT PPVLDSDGSFFLYSKLTVDKSRWQQGNVFSCSVMHEALHNHYTQKSLSLSPGK |
| 16 | anti-Fn EDA variant_H chain | Human | DNA | CAGGTGGAACTGGTGGAATCTGGCGGCGGATTGGTTCAGCCTGGCGGATCTCTGAGACTGTCT TGTGCCGCCTCCGGCTTCACCTTCAGCCTGTTCACAATGTCCTGGGTCCGACAGGCTCCTGGCA AAGGACTGGAATGGGTGTCCGCCATCTCTGGCAGCGGCGGCTCTACCTACTACGCCGATTCTGT GAAGGGCAGATTCACCATCAGCCGGGACAACTCCAAGAACACCCTGTACCTGCAGATGAACTC CCTGAGAGCCGAGGACACCGCCGTGTACTACTGTGCCAgaAGCACCCACCTGTATCTGTTCGAC TATTGGGGCCAGGGCACCCTGGTCACAGTGTCCTCTGCTTCTACCAAGGGACCCAGCGTGTTCC CTCTGGCTCCTTCCAGCAAGTCTACCTCTGGCGGAACAGCTGCTCTGGGCTGCCTGGTCAAGGA CTACTTTCCTGAGCCTGTGACCGTGTCCTGGAACTCTGGCGCTCTGACATCTGGCGTGCACACC TTTCCAGCTGTGCTGCAGTCCTCCGGCCTGTACTCTCTGTCCTCTGTCGTGACCGTGCCTTCCAG CTCTCTGGGAACCCAGACCTACATCTGCAATGTGAACCACAAGCCTTCCAACACCAAGGTGGA CAAGAAGGTGGAACCCAAGTCCTGCGACAAGACCCACACCTGTCCTCCATGTCCTGCTCCAGA ACTGCTCGGCGGACCTTCCGTGTTTCTGTTCCCTCCAAAGCCTAAGGACACCCTGATGATCTCT CGGACCCCTGAAGTGACCTGCGTGGTGGTGGATGTGTCTCACGAGGATCCCGAAGTGAAGTTC AATTGGTACGTGGACGGCGTGGAAGTGCACAACGCCAAGACCAAGCCTAGAGAGGAACAGTA CAACTCCACCTACAGAGTGGTGTCCGTGCTGACCGTGCTGCACCAGGATTGGCTGAACGGCAA AGAGTACAAGTGCAAGGTGTCCAACAAGGCCCTGCCTGCTCCTATCGAAAAGACCATCTCCAA GGCCAAGGGCCAGCCTAGGGAACCCCAGGTTTACACCTTGCCTCCATCTCGGGACGAGCTGAC CAAGAACCAGGTGTCCCTGACCTGTCTCGTGAAGGGCTTCTACCCCTCCGATATCGCCGTGGAA TGGGAGTCTAATGGCCAGCCTGAGAACAACTACAAGACAACCCCTCCTGTGCTGGACTCCGAC GGCTCATTCTTCCTGTACTCCAAGCTGACAGTGGACAAGTCCAGATGGCAGCAGGGCAACGTG TTCTCCTGCTCCGTGATGCACGAGGCCCTGCACAATCACTACACCCAGAAGTCCCTGTCTCTGT CCCCTGGCAAA |

EP 4 772 534 A1

[Table 8-10]

Table 8 (continued)

| | | | | |
|---|---|---|---|---|
| 17 | anti-Fn EDA variant_L chain | Human | Protein | DIVLTQSPATLSLSPGERATLSCRASQSVSMPFLAWYQQKPGQAPRLLIYGASSRATGVPARFSGSGSGTDFTLTISSLEPEDFATYYCQQMRGRPPTFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| 18 | anti-Fn EDA variant_L chain | Human | DNA | GACATCGTGCTGACCCAGTCTCCAGCCACACTGTCTCACTGTGTCTCCAGGCGAGAGAGCTACCCTGTCCTGTAGAGCCTCTCAGTCCGTGTCTATGCCCTTCCTGGCCTGGTATCAGCAGAAGCCTGGACAGGCTCCCCGGCTGTTGATCTACGGCGCTTCTTCTAGAGCTACCGGCGTGCCCGCTAGATTCTCCGGATCTGGCTCTGGCACCGACTTTACCCTGACCATCAGCTCCCTGGAACCTGAGGACTTCGCCACCTACTACTGCCAGCAGATGAGAGGCAGACCTCCTACCTTTGGCCAGGGCACCAAGGTGGAAATCAAGCGGACCGTGGCCGCTCCCTCCGTGTTCATCTTCCCACCTTCCGACGAGCAGTTGAAGTCCGGCACCGCTTCTGTGGTGTGCCTGCTGAACAACTTCTACCCCCGCGAGGCCAAGGTGCAGTGGAAGGTGGACAACGCCCTGCAGTCCGGCAACTCCCAGGAATCCGTGACCGAGCAGGACTCCAAGGACAGCACCTACTCCCTGTCCTCCACCCTGACCCTGTCCAAGGCCGACTACGAGAAGCACAAGGTGTACGCCTGCGAAGTGACCCACCAGGGCCTGTCTAGCCCCGTGACCAAGTCTTTCAACCGGGGCGAGTGC |
| 19 | anti-Fn EDA variant_H chain _L234A/ L235A | Human | Protein | QVELVESGGGLVQPGGSLRLSCAASGFTFSLFTMSWVRQAPGKGLEWVSAISGSGGSTYYADSVKGRFTISRDNSKNTLYLQMNSLRAEDTAVYYCARSTHLYLFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |

[Table 8-11]

Table 8 (continued)

| 20 | anti-Fn EDA variant_H chain _L234A/L 235A | Human | DNA | CAGGTGGAACTGGTGGAATCTGGCGGCGGATTGGTTCAGCCTGGCGGATCTCTGAGACTGTCTTG<br>TGCCGCCTCCGGCTTCACCTTCAGCCTGTTCACAATGTCCTGGGTCCGACAGGCTCCTGGCAAAG<br>GACTGGAATGGGTGTCCGCCATCTCTGGCAGCGGCGGCTCTACCTACTACGCCGATTCTGTGAAG<br>GGCAGATTCACCATCAGCCGGGACAACTCCAAGAACACCCTGTACCTGCAGATGAACTCCCTGA<br>GAGCCGAGGACACCGCCGTGTACTACTGTGCCAGAAGCACCCACCTGTATCTGTTCGACTATTGG<br>GGCCAGGGCACCCTGGTCACAGTGTCCTCTGCTTCTACCAAGGGACCCAGCGTGTTCCCTCTGGC<br>TCCTTCCAGCAAGTCTACCTCTGGCGGAACAGCTGCTCTGGGCTGCCTGGTCAAGGACTACTTTC<br>CTGAGCCTGTGACCGTGTCCTGGAACTCTGGCGCTCTGACATCTGGCGTGCACACCTTTCCAGCT<br>GTGCTGCAGTCCTCCGGCCTGTACTCTCTGTCCTCTGTCGTGACCGTGCCTTCCAGCTCTCTGGGA<br>ACCCAGACCTACATCTGCAATGTGAACCACAAGCCTTCCAACACCAAGGTGGACAAGAAGGTGG<br>AACCCAAGTCCTGCGACAAGACCCACACCTGTCCTCCATGTCCTGCTCCAGAAGCTGCTGGCGGC<br>CCTTCCGTGTTTCTGTTCCCTCCAAAGCCTAAGGACACCCTGATGATCTCTCGGACCCCTGAAGT<br>GACCTGCGTGGTGGTGGATGTGTCTCACGAGGATCCCGAAGTGAAGTTCAATTGGTACGTGGAC<br>GGCGTGGAAGTGCACAACGCCAAGACCAAGCCTAGAGAGGAACAGTACAACTCCACCTACAGA<br>GTGGTGTCCGTGCTGACCGTGCTGCACCAGGATTGGCTGAACGGCAAAGAGTACAAGTGCAAGG<br>TGTCCAACAAGGCCCTGCCTGCTCCTATCGAAAAGACCATCTCCAAGGCCAAGGGCCAGCCTAG<br>GGAACCCCAGGTTTACACCTTGCCTCCATCTCGGGACGAGCTGACCAAGAACCAGGTGTCCCTG<br>ACCTGTCTCGTGAAGGGCTTCTACCCCTCCGATATCGCCGTGGAATGGGAGTCTAATGGCCAGCC<br>TGAGAACAACTACAAGACAACCCCTCCTGTGCTGGACTCCGACGGCTCATTCTTCCTGTACTCCA<br>AGCTGACAGTGGACAAGTCCAGATGGCAGCAGGGCAACGTGTTCTCCTGCTCCGTGATGCACGA<br>GGCCCTGCACAATCACTACACCCAGAAGTCCCTGTCTCTGTCCCCTGGCAAA |
| 21 | anti-Fn EDA variant_H chain_E294Del | Human | Protein | QVELVESGGGLVQPGGSLRLSCAASGFTFSLFTMSWVRQAPGKGLEWVSAISGSGGSTYYADSVKG<br>RFTISRDNSKNTLYLQMNSLRAEDTAVYYCARSTHLYLFDYWGQGTLVTVSSASTKGPSVFPLAPSS<br>KSTSGGTAALGCLVKDYFPEPVTVSWNSGALTSGVHTFPAVLQSSGLYSLSSVVTVPSSSLGTQTYIC<br>NVNHKPSNTKVDKKVEPKSCDKTHTCPPCPAPELLGGPSVFLFPPKPKDTLMISRTPEVTCVVVDVSH<br>EDPEVKFNWYVDGVEVHNAKTKPREQYNSTYRVVSVLTVLHQDWLNGKEYKCKVSNKALPAPIEK<br>TISKAKGQPREPQVYTLPPSRDELTKNQVSLTCLVKGFYPSDIAVEWESNGQPENNYKTTPPVLDSDG<br>SFFLYSKLTVDKSRWOOGNVFSCSVMHEALHNHYTOKSLSLSPGK |

EP 4 772 534 A1

[Table 8-12]

Table 8 (continued)

| | | | |
|---|---|---|---|
| 22 | anti-Fn EDA variant_H chain_E294Del | Human | DNA | CAGGTGGAACTGGTGGAATCTGGCGGCGGATTGGTTCAGCCTGGCGGATCTCTGCGGCTGTCTTGTGCCGCCTCCGGCTTCACCTTCAGCCTGTTCACAATGTCCTGGGTCCGACAGGCTCCTGGCAAAGGACTGGAATGGGTGTCCGCCATCTCTGGCAGCGGCGGCTCTACCTACTACGCCGATTCTGTGAAGGGCAGATTCACCATCAGCCGGGACAACTCCAAGAACACCCTGTACCTGCAGATGAACTCCCTGAGAGCCGAGGAGGACACCGCCGTGTACTACTGTGCCgaaAGCACCCACCTGTATCTGTTCGACTATTGGGGCCAGGGCACCCTGGTCACAGTGTCTCGCTTCTCTACCAAGGGACCCAGCGTGTTCCCTCTGGCTCCTTCCAGCAAGTCTACCTCTGGCGGAACAGCTGCTGTGCTGGGCTGTCTGGTCAAGGACTACTTTCCTGAGCCTGTGACCGTGTCCTGGAACTCTGGCGCTCTGACCAGCGGCGTGCACACCTTCCCAGCTGTGCTGCAGTCCTCCGGCCTGTACTCTCTCTGTGTGACCGTGCCTTCCAGCTCTCTGGGGAACCCAGACCTACATCTGCAACGTGAATCACAAGCCTTCCAACACCACCAAGGTGGACAAGAAGGTGGAACCCAAGTCCTGCGACAAGACCCACACCTGTCCTCCATGTCCTGCTCCAGAACCTGCTGGGCGGACCTTCCGTGTTCTGTTCCCTCCAAAGCCTAAGGACACCCTGATGATCTCTCGGACCCCTGAAGTGACCTGCGTGGTGGTGGATGTGTCTCACGAGGATCCGGAAGTTCAATTGGTACGTGGACGGCGTGGAAGTGCACAACGCCAAGACCAAGCCTAGAGAGGAGCAGTACAACTCCACCTACAGAGTGGTGTCCGTGCTGACCGTGCTGCACCAGGATTGGCTGAACGGCAAAGAGTACAAGTGCAAGGTGTCCAACAAGGCCCTGCCTGCTCCTATCGAAAAGACCATCTCCAAGGCCAAGGGCCAGCCTAGGGAACCCCAGGTTACACCTTGCCTGCCTCCTGGACGAGCTGACCAAGAACCAGGTGTCCCTGACCTGTCTCGTGAAGGGCTTCTACCCCTCCGATATCGCCGTGGAATGGGAGTCTAATGGCCAGCCTGAGAACAACTACAAGACAACCCCTCCTGTGCTGGACTCCGACGGCTCATTCTTCCTGTACTCCAAGCTGACAGTGGACAAGTCCAGATGGCAGCAGGGCAACGTGTTCTCCTGCTCCGTGATGCACGAGGCCCTGCACAATCACTACACCCAGAAGTCCCTGTCTCTGTCCCCTGGCAAA |
| 23 | anti-Fn EDA variant_H chain_L234A/L235A/E294Del | Human | Protein | QVELVESGGGLVQPGGSLRLSCAASGFTFSLFTMSWVRQAPGKGLEWVSAISGSGGSTYYADSVKGRFTISRDNSKNTLYLQMNSLRAEDTAVYYCARSTHLYLFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEAAGGPSVFLFPPKPKDTLMISRTPEVTCVVVDVSHEDPEVKFNWYVDGVEVHNAKTKPREQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |

[Table 8-13]

| Table 8 (continued) | | | | |
|---|---|---|---|---|
| 24 | anti-Fn EDA variant_H chain_L234A/ L235A/E294D el | Human | DNA | CAGGTGGAACTGGTGGAATCTGGCGGAGGATTGGTTCAGCCTGGCGGCTCTCTGAGAC TGTCTTGTGCCGCCTCTGGCTTCACCTTCAGCCTGTTCACCATGTCCTGGGTCCGACAGG CTCCTGGCAAAGGACTGGAATGGGTGTCCGCCATCTCTGGCTCTGGCGGCAGCACCTAC TACGCCGATTCTGTGAAGGGCAGATTCACCATCAGCCGGGACAACTCCAAGAACACCC TGTACCTGCAGATGAACTCCCTGAGAGCCGAGGACACCGCCGTGTACTACTGTGCCAga AGCACCCACCTGTATCTGTTCGACTATTGGGGCCAGGGCACCCTGGTCACAGTCTCTTC TGCTTCTACCAAGGGACCCAGCGTGTTCCCTCTGGCTCCTAGCTCTAAGTCCACCTCTG GCGGAACAGCTGCTCTGGGCTGTCTGGTCAAGGACTACTTCCCTGAGCCTGTGACCGTG TCTTGGAACTCTGGCGCTCTGACATCCGGCGTGCACACCTTTCCAGCTGTGCTGCAATC CTCCGGCCTGTACTCTCTGTCCTCCGTCGTGACCGTGCCTTCTAGCTCTCTGGGCACCCA GACCTACATCTGCAATGTGAACCACAAGCCTTCCAACACCAAGGTGGACAAGAAGGTG GAACCCAAGTCCTGCGACAAGACCCACACCTGTCCTCCATGTCCTGCTCCAGAAGCTGC TGGCGGCCCTTCCGTGTTTCTGTTCCCTCCAAAGCCTAAGGACACCCTGATGATCTCTC GGACCCCTGAAGTGACCTGCGTGGTGGTGGATGTGTCTCACGAGGACCCAGAAGTGAA GTTCAATTGGTACGTGGACGGCGTGGAAGTGCACAACGCCAAGACCAAGCCTAGAGAG CAGTACAACTCCACCTACAGAGTGGTGTCCGTGCTGACCGTGCTGCACCAGGATTGGCT GAACGGCAAAGAGTACAAGTGCAAGGTGTCCAACAAGGCCCTGCCTGCTCCTATCGAA AAGACCATCTCCAAGGCCAAGGGCCAGCCTAGGGAACCCCAGGTTTACACCTTGCCTC CATCTCGGGACGAGCTGACCAAGAACCAGGTGTCCCTGACCTGTCTCGTGAAGGGCTT CTACCCCTCCGATATCGCCGTGGAATGGGAGTCTAATGGCCAGCCAGAGAACAACTAC AAGACAACCCCTCCTGTGCTGGACTCCGACGGCTCATTCTTCCTGTACTCCAAGCTGAC AGTGGACAAGTCCAGATGGCAGCAGGGCAACGTGTTCTCCTGCTCCGTGATGCACGAG GCCCTGCACAATCACTACACACAGAAGTCCCTGTCTCTGTCCCCTGGCAAG |

Although some embodiments of the present invention are described above, these embodiments are presented as examples and do not intend to limit the scope of the invention. These novel embodiments may be carried out in other various forms, and various omissions, replacements, and changes or modifications can be made therein without departing from the gist of the invention. These embodiments or their modifications are included in the scope and the gist of the invention and also included in the scope of aspects of the invention described in claims and a scope equivalent thereto.

Industrial Applicability

The sialylated Fc region variant with a further increased amount of sialic acid added according to the present invention is useful in the prevention or treatment of various autoimmune diseases and inflammatory diseases.

**Claims**

1.  A sialylated Fc region variant comprising the following amino acid variations (1) and (2):

    (1)

    (a) amino acid residues 234 and/or 235 are substituted by serine, threonine, asparagine, glutamine, cysteine, aspartic acid, glutamic acid, lysine, arginine, histidine, glycine, alanine, proline, methionine, valine, or isoleucine, wherein the amino acids at positions 234 and 235 are the same or different after substitution; (b) amino acid residue 234 is substituted by phenylalanine, tyrosine, tryptophane, isoleucine, valine, methionine, proline, alanine, or glycine, and amino acid residue 235 is substituted by serine, threonine, asparagine, glutamine, cysteine, aspartic acid, glutamic acid, lysine, arginine, or histidine; or (c) amino acid residue 234 is substituted by serine, threonine, asparagine, glutamine, cysteine, aspartic acid, glutamic acid, lysine, arginine, or histidine, and amino acid residue 235 is substituted by phenylalanine, tyrosine, tryptophane, isoleucine, valine, methionine, proline, alanine, or glycine; and

    (2) amino acid residue 293 or 294 is deleted, wherein the amino acid residue numbers are amino acid positions according to EU index.

2.  The sialylated Fc region variant according to claim 1, wherein amino acid residues 234 and 235 are substituted by serine, threonine, asparagine, glutamine, cysteine, aspartic acid, glutamic acid, lysine, arginine, histidine, glycine, alanine, proline, methionine, valine, or isoleucine, wherein the amino acids at positions 234 and 235 are the same or different after substitution.

3.  The sialylated Fc region variant according to claim 1 or 2, wherein

    amino acid residues 234 and 235 are substituted by alanine, or amino acid residue 234 is substituted by alanine, and amino acid residue 235 is substituted by glutamic acid.

4.  The sialylated Fc region variant according to any one of claims 1 to 3, wherein the Fc region is derived from human IgG.

5.  The sialylated Fc region variant according to claim 4, wherein the Fc region is derived from human Igγ1, human Igy2, human Igγ3, or human Igγ4.

6.  The sialylated Fc region variant according to claim 4, wherein the sialylated Fc region variant comprises an amino acid sequence 90% or more identical to a sequence consisting of amino acids at positions 218 to 448 in the amino acid sequence represented by SEQ ID NO: 7 or an amino acid sequence 90% or more identical to a sequence consisting of amino acids at positions 217 to 447 in the amino acid sequence represented by SEQ ID NO: 13.

7.  A polypeptide comprising the sialylated Fc region variant according to any one of claims 1 to 6.

8.  The polypeptide according to claim 7, wherein the polypeptide comprising the sialylated Fc region variant is an antibody.

9.  The polypeptide according to claim 8, wherein the antibody is an IgG antibody.

**10.** The polypeptide according to claim 7, wherein the polypeptide comprising the sialylated Fc region variant is an Fc fusion protein molecule.

**11.** The polypeptide according to claim 10, wherein the sialylated Fc fusion protein molecule comprises a non-human derived protein.

**12.** A polynucleotide encoding the polypeptide according to any one of claims 7 to 11.

**13.** An expression vector comprising the polynucleotide according to claim 12.

**14.** A host cell transformed with the expression vector according to claim 13.

**15.** A method for producing a polypeptide comprising a sialylated Fc region variant, the method comprising the step of culturing the host cell according to claim 14.

**16.** The method according to claim 15, further comprising the step of adding or expressing β-galactoside α-2,6-sialyltransferase 1 (ST6GAL1).

**17.** An Fc region variant comprising the following amino acid variations (1) and (2):

(1)

(a) amino acid residues 234 and/or 235 are substituted by serine, threonine, asparagine, glutamine, cysteine, aspartic acid, glutamic acid, lysine, arginine, histidine, glycine, alanine, proline, methionine, valine, or isoleucine, wherein the amino acids at positions 234 and 235 are the same or different after substitution;
(b) amino acid residue 234 is substituted by phenylalanine, tyrosine, tryptophane, isoleucine, valine, methionine, proline, alanine, or glycine, and amino acid residue 235 is substituted by serine, threonine, asparagine, glutamine, cysteine, aspartic acid, glutamic acid, lysine, arginine, or histidine; or
(c) amino acid residue 234 is substituted by serine, threonine, asparagine, glutamine, cysteine, aspartic acid, glutamic acid, lysine, arginine, or histidine, and amino acid residue 235 is substituted by phenylalanine, tyrosine, tryptophane, isoleucine, valine, methionine, proline, alanine, or glycine; and

(2) amino acid residue 293 or 294 is deleted,
wherein the amino acid residue numbers are amino acid positions according to EU index.

**18.** A pharmaceutical composition comprising the polypeptide according to any one of claims 7 to 11 and a pharmaceutically acceptable carrier.

**19.** The pharmaceutical composition according to claim 18 for use in the treatment of an autoimmune disease.

**20.** The polypeptide according to any one of claims 7 to 11 for use in the treatment of an autoimmune disease.

**21.** A method for treating an autoimmune disease, comprising the step of administering a therapeutically effective amount of the polypeptide according to any one of claims 7 to 11 to a subject.

**22.** Use of the polypeptide according to any one of claims 7 to 11 in the production of a pharmaceutical composition for the treatment of an autoimmune disease.

Fig. 1

Full-length structure of wild-type IgG1 (PDB:1HZH)

Fig. 2

N297 (E294Del)

Loop at N297 (E294Del)

Crystal structure of wild-type IgG1 (WT)
Sialylated E294Del model structure (E294Del)

Fig. 3

Sialylated E294Del
model structure

Fig. 4

Sialylated
L234A/L235A/
E294Del model
structure

Fig. 5

Sialylated
L234A/L235E/
E294Del model
structure

Fig. 6

Sialylated
L234F/L235Q/
E294Del model
structure

## INTERNATIONAL SEARCH REPORT

| | International application No. |
|---|---|
| | **PCT/JP2024/030889** |

| A. | CLASSIFICATION OF SUBJECT MATTER |
|---|---|

*C07K 16/00*(2006.01)i; *A61K 39/395*(2006.01)i; *A61K 47/54*(2017.01)i; *A61P 37/02*(2006.01)i; *C12N 1/15*(2006.01)i; *C12N 1/19*(2006.01)i; *C12N 1/21*(2006.01)i; *C12N 5/10*(2006.01)i; *C12N 15/13*(2006.01)i; *C12N 15/63*(2006.01)i; *C12P 21/08*(2006.01)i

FI: C07K16/00; A61K39/395 D; A61K39/395 N; A61K39/395 V; A61K47/54; A61P37/02; C12N1/15; C12N1/19; C12N1/21; C12N5/10; C12N15/13 ZNA; C12N15/63 Z; C12P21/08

According to International Patent Classification (IPC) or to both national classification and IPC

| B. | FIELDS SEARCHED |
|---|---|

Minimum documentation searched (classification system followed by classification symbols)

C07K16/00; A61K39/395; A61K47/54; A61P37/02; C12N1/15; C12N1/19; C12N1/21; C12N5/10; C12N15/13; C12N15/63; C12P21/08

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | WO 2022/233320 A1 (INNOVENT BIOLOGICS (SUZHOU) CO., LTD.) 10 November 2022 (2022-11-10) p. 2, lines 14-22, examples, claims | 1-22 |
| Y | JP 2014-519836 A (LABORATOIRE FRANCAIS DU FRACTIONNEMENT ET DES BIOTECHNOLOGIES) 21 August 2014 (2014-08-21) claims, examples | 1-22 |
| Y | 大海雄介ほか, 自己免疫疾患とシアル酸転移酵素, 生化学, 2017, vol. 89, line 5, pp. 666-672, (OHMI, Yuhsuke et al., Sialyltransferases in autoimmune diseases, Journal of Japanese Biochemical Society) "1. Introduction", "4. Glycans on ACPA", fig. 2 | 1-16, 18-22 |

☑ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **07 October 2024** | **22 October 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

| International application No. |
|---|
| **PCT/JP2024/030889** |

**C.    DOCUMENTS CONSIDERED TO BE RELEVANT**

| Category* | Citation of document, with indication, where appropriate, of the relevant passages | Relevant to claim No. |
|---|---|---|
| A | WO 2014/163101 A1 (CHUGAI SEIYAKU KABUSHIKI KAISHA) 09 October 2014 (2014-10-09)<br>examples, claims | 1-22 |

Form PCT/ISA/210 (second sheet) (July 2022)

**INTERNATIONAL SEARCH REPORT**

International application No.

**PCT/JP2024/030889**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/JP2024/030889**

| Patent document cited in search report | | | Publication date (day/month/year) | Patent family member(s) | | | Publication date (day/month/year) |
|---|---|---|---|---|---|---|---|
| WO | 2022/233320 | A1 | 10 November 2022 | US | 2024/0228579 | A1 | |
| | | | | paragraph [0007], examples, claims | | | |
| | | | | JP | 2024-516320 | A | |
| | | | | EP | 4335868 | A1 | |
| | | | | CN | 117295760 | A | |
| | | | | CA | 3218187 | A1 | |
| | | | | AU | 2022269145 | A1 | |
| | | | | TW | 202246313 | A | |
| JP | 2014-519836 | A | 21 August 2014 | US | 2014/0378663 | A1 | |
| | | | | examples, claims | | | |
| | | | | WO | 2012/175751 | A2 | |
| | | | | EP | 2537864 | A1 | |
| | | | | CA | 2840127 | A1 | |
| | | | | CN | 103827142 | A | |
| | | | | BR | 112013033342 | A2 | |
| | | | | ES | 2755708 | T3 | |
| WO | 2014/163101 | A1 | 09 October 2014 | US | 2016/0039912 | A1 | |
| | | | | examples, claims | | | |
| | | | | EP | 2982689 | A1 | |
| | | | | AU | 2014250434 | A1 | |
| | | | | CA | 2908350 | A1 | |
| | | | | KR | 10-2015-0136514 | A | |
| | | | | CN | 105246914 | A | |
| | | | | MX | 2015014017 | A | |
| | | | | HK | 1215034 | A1 | |
| | | | | RU | 2015146769 | A | |
| | | | | BR | 112015024587 | A2 | |
| | | | | SG | 11201508170T | A | |
| | | | | TW | 201529599 | A | |

Form PCT/ISA/210 (patent family annex) (July 2022)

## REFERENCES CITED IN THE DESCRIPTION

### Patent documents cited in the description

- WO 2012113863 A
- JP 2017522040 W
- US 5225539 A
- US 6180370 B
- WO 9007861 A
- WO 2012175751 A

### Non-patent literature cited in the description

- *Journal of Allergy and Clinical Immunology*, March 2017, vol. 139 (3), S1-S46
- *Science*, 04 August 2006, vol. 313 (5787), 670-673
- *Blood*, 09 September 2010, vol. 116 (10), 1698-1704
- **KABAT et al.** Sequences of Proteins of Immunological Interest. 1991
- *Nucleic Acids Res.*, 2015, vol. 43, W580-W584
- *J Mol Biol.*, 1982, vol. 157 (1), 105-32
- *Mol. Immunol*, 1992, vol. 29, 633-639
- *Proc. Natl. Acad. Sci. USA*, 2006, vol. 103 (11), 4005-4010
- *Nature Biotech*, 1998, vol. 16, 677-681
- *J. Mol. Biol.*, 1997, vol. 270, 26-35
- *Proc. Natl. Acad. Sci. USA*, 2013, vol. 110, E2987-E2996
- *Science*, 1959, vol. 130, 432-437
- *Virol*, 1959, vol. 8, 396
- *J. Am. Med. Assoc*, 1967, vol. 199, 519
- *Exp. Biol. Med*, 1950, vol. 73, 1-8
- *Proc. Natl. Acad. Sci. USA.*, 1985, vol. 82, 8404
- Molecular Cloning. Cold Spring Harbor Laboratory, vol. 3, A2
- *Proc. Natl. Acad. Sci. USA.*, 1980, vol. 77, 4505
- *CELL IMMUNOL.*, 2000, vol. 200 (1), 16-26
- *MAbs*, 2015, vol. 7 (3), 571-83
- *J. Biol. Chem.*, 2017, vol. 292 (9), 3900-3908
- *Nature*, 2000, vol. 406, 267-273